(19) Europäisches Patentamt
European Patent Office
Office européen des brevets

(11) **EP 3 404 667 B1**

(12) **EUROPEAN PATENT SPECIFICATION**

(45) Date of publication and mention
of the grant of the patent:
**28.02.2024   Bulletin 2024/09**

(21) Application number: **18172539.1**

(22) Date of filing: **16.05.2018**

(51) International Patent Classification (IPC):
*G16H 50/50* (2018.01)   *G16H 50/70* (2018.01)
*G16H 15/00* (2018.01)   *G16H 50/20* (2018.01)
*G16H 30/40* (2018.01)

(52) Cooperative Patent Classification (CPC):
**G16H 50/50; G16H 15/00; G16H 30/40;**
**G16H 50/20; G16H 50/70;** G16H 20/10;
G16H 50/30; Y02A 90/10

(54) **LEARNING BASED METHODS FOR PERSONALIZED ASSESSMENT, LONG-TERM PREDICTION AND MANAGEMENT OF ATHEROSCLEROSIS**

LERNBASIERTE VERFAHREN ZUR PERSONALISIERTEN BEURTEILUNG, LANGZEITVORHERSAGE UND VERWALTUNG VON ATHEROSKLEROSE

PROCÉDÉS À BASE D'APPRENTISSAGE POUR L'ÉVALUATION PERSONNALISÉE, LA PRÉDICTION À LONG TERME ET LA GESTION DE L'ATHÉROSCLÉROSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2017   US 201715599505**
**19.05.2017   EP 17464007**

(43) Date of publication of application:
**21.11.2018   Bulletin 2018/47**

(73) Proprietor: **Siemens Healthineers AG**
**91301 Forchheim (DE)**

(72) Inventors:
• **Itu, Lucian Mihai**
**500294 Brasov (RO)**
• **Passerini, Tiziano**
**Plainsboro, NJ New Jersey 08536 (US)**
• **Rapaka, Saikiran**
**Pennington, NJ New Jersey 08534 (US)**
• **Sharma, Puneet**
**Princeton Junction, NJ New Jersey 08550 (US)**

• **Comaniciu, Dorin**
**Princeton Junction, NJ New Jersey 08550 (US)**

(74) Representative: **Bals & Vogel Patentanwälte PartGmbB**
**Sendlinger Strasse 42A**
**80331 München (DE)**

(56) References cited:
**WO-A1-2015/058044      WO-A2-2016/075331**
**US-A1- 2016 148 371      US-A1- 2016 310 018**
**US-A1- 2017 018 081**

• **ALBERTO FIGUEROA C ET AL: "A computational framework for fluid-solid-growth modeling in cardiovascular simulations", COMPUTER METHODS IN APPLIED MECHANICS AND ENGINEERING, NORTH-HOLLAND, AMSTERDAM, NL, vol. 198, no. 45-46, 15 September 2009 (2009-09-15), pages 3583-3602, XP026601482, ISSN: 0045-7825, DOI: 10.1016/J.CMA.2008.09.013 [retrieved on 2008-10-18]**

## Description

### TECHNICAL FIELD

The present invention relates generally to methods for the personalized evaluation of atherosclerotic plaques using machine-learning techniques.

### BACKGROUND

Cardiovascular disease (CVD) is the leading cause of death, globally. The most prevalent and devastating CVD is atherosclerosis, a chronic, inflammatory, fibroproliferative disorder primarily of large- and medium-sized conduit arteries. Its initiation and progression is closely linked to the vascular endothelium, which responds to the dynamic forces acting on the vessel wall because of the complex geometry of the arteries, and the acquired presence of pathologic characteristics such as atherosclerotic plaque.

The majority of cardiovascular events, like stroke and myocardial infarction, are caused by atherosclerotic plaque ruptures, which cause distal embolization. Previous studies have shown that hemodynamic quantities are linked to the initiation, progression and rupture of atherosclerotic plaques. These hemodynamic quantities are in turn influenced both by systemic properties and the local geometry (certain regions, like bifurcations are predisposed to plaque formation). Thus, hemodynamic quantities, i.e. pressure, velocities, flow rate and flow-generated endothelial shear stresses (ESS), play a crucial role in the understanding of plaque initiation, progression, and rupture. Further, a combination of hemodynamic factors with other patient information (like demographics, blood biomarker information, past history, etc.) can be used to obtain a coherent understanding of the patient's condition as well as better understanding of patient outcomes.

US 2016/148371 A1 discloses a method for computation of blood flow in a vessel of a patient. In particular, a hemodynamic metric is estimated from non-invasive medical imaging data. As another example, US 2017/018081 A1 shows non-invasive cardiovascular disease assessment, treatment planning, and related methods. Also, methods for non-invasively assessing blood flow characteristics using measurements and estimates of contrast distribution are disclosed None of the above prior art documents discloses performing fluid solid growth (FSG) computations on in silico anatomical models for the purpose of assessment of atherosclerotic plaque.

### SUMMARY

Embodiments of the present invention address and overcome one or more of the above shortcomings and drawbacks by providing methods related to the personalized assessment of atherosclerotic plaque using machine-learning techniques.

According to some embodiments, a computer-implemented method for providing a personalized evaluation of assessment of atherosclerotic plaques for a patient acquires patient data comprising non-invasive patient data, medical images of the patient, and blood biomarkers. Features of interest are extracted from the patient data a data driven surrogate model is applied to the features of interest to predict one or more measures of interest related to atherosclerotic plaque. In some embodiments, a report is generated in a structured format describing the measures of interest related to atherosclerotic plaque. This report may be stored in a patient-specific medical record.

In a method according to the invention, the data driven surrogate model is trained for predicting measures of interest using a machine learning method, wherein the machine learning models are trained using a database of synthetic data comprising one or more of *in silico* anatomical models. Some embodiments may also comprise *in vitro* anatomical models. The machine learning models are trained by first performing fluid solid growth (FSG) computations for the *in silico* anatomical models. Geometric features and plaque-related features are extracted from the database of synthetic data. Then, the machine learning models are trained to predict measures of interest related to atherosclerotic plaque using the measures of interest from the geometric features, and the plaque-related features.

The *in silico* anatomical models are generated by initializing a new *in silico* anatomical model skeleton of a coronary arterial tree anatomical model by prescribing a number of vessels at each generation of the coronary arterial tree and defining healthy geometric information for each generation of the coronary arterial tree. The healthy geometric information comprises one or more of vessel radius, a degree of tapering, and a branch length. Stenoses in the coronary arterial tree are then established, which modify the healthy geometric information. Additionally, plaque composition is established for each stenosis in the coronary arterial tree. Next, the new *in silico* anatomical model is updated based on the modified healthy geometric information and the plaque composition for each stenosis in the coronary arterial tree. Once updated, the new *in silico* anatomical model is stored in the database of synthetic data.

In some embodiments, the plaque composition for each stenosis may be established by randomly selecting a particular plaque composition from a plurality of predefined plaque composition types. For example, in one embodiment, the plaque composition for each stenosis comprises a plaque material established by (i) randomly selecting a center of each volume of plaque material; (ii) randomly selecting a size and a shape for the plaque material; (iii) randomly selecting material proprieties for the plaque composition; and (iv) establishing the plaque material using the center, size, shape, and material properties. In one embodiment, the plaque composition for each stenosis may mimic a predefined high risk plaque composition.

In some embodiments, multiple machine learning models may be used with the aforementioned method. For example, in one embodiments, the machine learning models may comprise a first machine model trained to predict plaque formation, a second machine model trained to predict plaque development, and a third machine model trained to predict plaque rupture. In other embodiments, the machine learning models may include a first machine learning model trained to predict an ischemic weight of each branch, a second machine learning model trained to predict ischemic contribution scores, a third machine learning model trained to predict hemodynamic measures of interest, a fourth machine learning model trained to predict plaque related measures of interest, and a fifth machine learning model trained to predict a risk of future cardiovascular event. Where multiple machine learning models are employed, they may be applied in parallel to the features of interest in some embodiments. In some embodiments, the machine learning models may be applied in a cascaded workflow that sequentially applies the machine learning models using outputs of each machine learning model as inputs for a subsequent machine learning model in the cascaded workflow.

Some embodiments of the aforementioned method may further include visualization aspects. For example, in one embodiment, a visualization may be generated depicting the measures of interest related to atherosclerotic plaque. In some embodiments, the visualization may comprise a coronary artery image and, in response to user selection of a location within the coronary artery image, a measure of interest corresponding to the location is presented in the visualization.

According to another aspect of the present invention, a computer-implemented method for training a machine learning model to provide a personalized evaluation of assessment of atherosclerotic plaques for a patient includes generating a database of synthetic data comprising one or more of *in silico* anatomical models and performing FSG computations for the *in silico* anatomical models to yield output data. The method further includes extracting measures of interest from the output data, as well as geometric features of the *in silico* anatomical models and plaque-related features from the database of synthetic data. Machine learning models are trained to generate predicted measurements related to atherosclerotic plaque based on the geometric features of the *in silico* anatomical models trees and the plaque-related features.

Additional features and advantages of the invention will be made apparent from the following detailed description of illustrative embodiments that proceeds with reference to the accompanying drawings.

## BRIEF DESCRIPTION OF THE DRAWINGS

The foregoing and other aspects of the present invention are best understood from the following detailed description when read in connection with the accompanying drawing. For the purpose of illustrating the invention, there are shown in the drawing exemplary embodiments that are presently preferred, it being understood, however, that the invention is not limited to the specific embodiments disclosed. Included in the drawings are the following Figures:

FIG. 1 shows a workflow used to predict measures of interest related to the formation, progression and rupture of plaque in the human cardiovascular system, according to some embodiments;
FIG. 2 shows a workflow used to train one or more data-driven surrogate models for predicting measures of interest related to the formation, progression and rupture of plaque in the human cardiovascular system, not forming part of the claimed invention;
FIG. 3 shows a workflow for computing patient-specific plaque related measures of interest, according to some embodiments;
FIG. 4A presents a visualization of a method for generating synthetic *in silico* pathologic coronary geometries, according to some embodiments;
FIG. 4B presents a visualization of the first step of the method shown in FIG. 4A;
FIG. 4C presents a visualization of the second step of the method shown in FIG. 4A;
FIG. 4D presents a visualization of the third step of the method shown in FIG. 4A;
FIG. 4E presents a visualization of the fourth step of the method shown in FIG. 4A;
FIG. 5 illustrates a parallel machine learning based prediction of plaque formation, development and rupture, according to some embodiments;
FIG. 6 shows a cascaded machine learning based workflow for the prediction of plaque related measures of interest, according to some embodiments;
FIG. 7 illustrates an example workflow for estimating the uncertainty of plaque related measures of interest metric using a machine learning (ML) model, according to some embodiments;
FIG. 8 presents a technique to retraining an ML model based on patient-specific cases with bad match between predicted and measured plaque related metric of interest, according to some embodiments;
FIG. 9 illustrates a workflow for training an ML model on in vitro data for predicting plaque related measures of interest, not forming part of the claimed invention;
FIG. 10 shows an example in vitro model used for generating the training database not forming part of the claimed invention;
FIG. 11 shows a workflow for training an ML model on in silico data generated from a physics based model derived from in vitro data, not forming part of the claimed invention;

FIG. 12 illustrates an atherosclerotic plaque with main constituents and plaque appearance on a Computed Tomography Angiography (CTA) image;

FIG. 13 presents a workflow for training an ML model to perform image-based plaque histology, not forming part of the claimed invention;

FIG. 14 shows a workflow for training ML models for identifying plaques on medical images, not forming part of the claimed invention;

FIG. 15 shows a workflow for performing individual lesion assessment, not forming part of the claimed invention;

FIG. 16 presents an ML based workflow for predicting the effect of a treatment plan, not forming part of the claimed invention;

FIG. 17 shows examples of a tool to point and click points on an image, where the system visualizes the point and the associated measure of interest;

FIG. 18 provides an example visualization of coronary plaque at present and future dates; and

FIG. 19 provides an example of a parallel processing memory architecture that may be utilized to implement the ML models and other aspects of the various workflows discussed herein.

DETAILED DESCRIPTION

The following disclosure describes the present invention according to several embodiments directed at methods related to the personalized assessment of atherosclerotic plaque using machine learning (ML) techniques. More specifically, the techniques described herein use ML models to predict measures of interest related to plaque such as risk of a cardiovascular event, indication of a future screening date, plaque composition (absolute / relative values of plaque components), plaque evolution related measure of interest (future size, shape, composition and location of plaques), in-stent restenosis, lesions requiring sealing, therapy planning, etc. The prediction is based on various features extracted from non-invasive patient data, medical imaging data (e.g., noninvasive imaging (Computed Tomography (CT), Echocardiography (stress/rest, with/without contrast agent), Magnetic Resonance Imaging (MRI), etc.), invasive imaging (X-ray angiography, IVUS, OCT, NIRS)), blood biomarkers, radiogenomic information, wearable sensors, etc. One advantage of the ML based workflows discussed herein is that data from heterogeneous sources may be integrated to perform a comprehensive assessment.

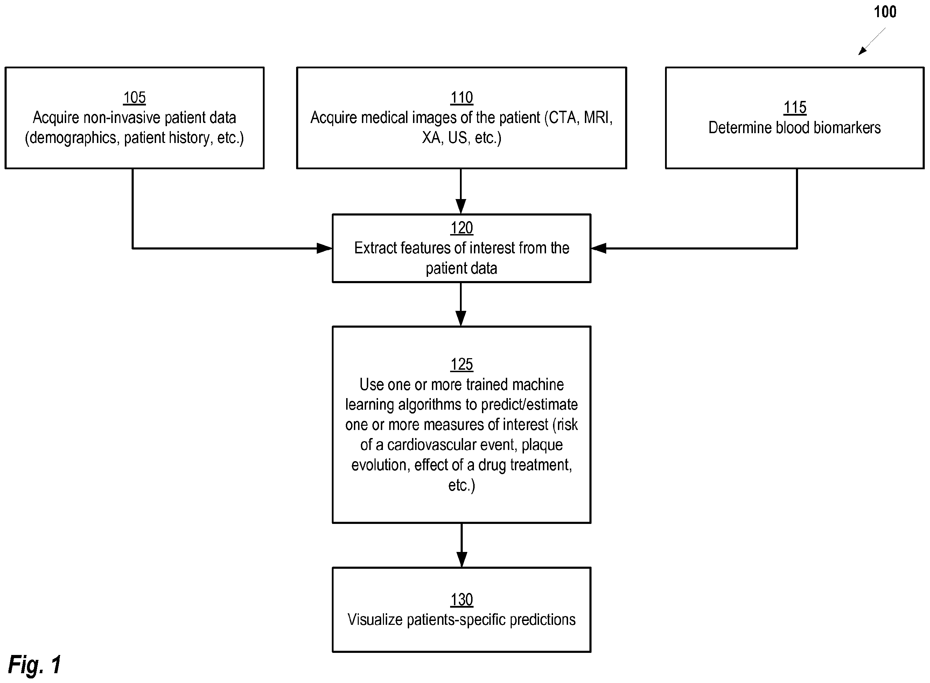

FIG. 1 illustrates a workflow 100 for predicting measures of interest related to the formation, progression, and rupture of plaque in the human cardiovascular system, according to some embodiments. Starting at step 105, non-invasive patient data is acquired. The term "non-invasive patient data" refers to any data that may be acquired using non-invasive techniques. Examples of non-invasive patient data that may be acquired at step 105 include, without limitation, demographics and patient history. The non-invasive patient data can be acquired via a wide range of sensors, comprising medical equipment and devices (stethoscope, blood pressure meter, imaging scanners, laboratory diagnostic, etc. ) as well as non-medical grade devices (including, without limitation, wearables) for the measurements of physiological signals. In some embodiments, the non-invasive patient data includes biochemical signals as produced, for example, by blood tests and molecular measurements (e.g., "omics" such as proteomics, transcriptomics, genomics, metabolomics, lipidomics, and epigenomics). Additionally, the non-invasive patient data may span a wide range of biometrics signals, and can be driven by the individual as in the Quantified Self movement, promoting self-monitoring and self-sensing through wearable sensors and wearable computing. Furthermore, features extracted based on radio-genomics may be generated and used as non-invasive patient data (e.g., imaging biomarkers that are linked with the genomics of a pathology).

At step 110, medical images of the patient are acquired. Generally, any medical imaging technique known in the art may be used in acquiring the images at step 110. Thus, non-invasive imaging techniques may be applied at step 110 such as MRI, Computed Tomography (CT), Echocardiography (stress/rest, with/without contrast agent), as well as invasive imaging techniques such as X-ray angiography, Intravascular ultrasound (IVUS), Optical Coherence Tomography (OCT), and Near-infrared Spectroscopy (NIRS).

At step 115, blood biomarkers are determined based on blood measurements of the patient. These blood biomarkers may include, for example, Complement Reactive Protein, Fibrinogen, White Blood Cell Count, IL-6, IL-18 & TNF-$\alpha$, Circulating Soluble CD40 Ligand, Vascular Calcification Markers (osteopontin, osteoprotegerin, etc.), Matrix Metalloproteinases, Myeloperoxidase, Platelet-derived growth factors, cardiac troponin I, cardiac troponin T, creatine kinase, creatine kinase myocardial band, total cholesterol, level of serum C-reactive protein, C-reactive protein, lactate dehydrogenase (LDH), aspartate transaminase (AST), myoglobin (Mb), ischemia-modified albumin (IMA), glycogen phosphorylase isoenzyme BB, High-density lipoprotein (HDL) cholesterol, low-density lipoprotein (LDL) cholesterol, pregnancy associated plasma protein A (PAPP-A), insulin-like growth factor binding protein-4 (IGFBP-4) and its fragments, myeloperoxidase (MPO), fatty acid binding protein (FABP), troponin C (TnC), D-dimer and high molecular weight fibrin degradation products, soluble CD40 ligand (sCD40L), cystatin C, human serum albumin (HSA), procalcitonin (PCT) , glycogen phosphorylase isoenzyme BB (GPBB), serum amyloid A (SAA), retinolbinding protein 4 (RBP4), soluble lectin-like oxidized LDL receptor (sLOX-1), adiponectin (Adn), and the S100 protein.

The different types of information acquired at steps 105 - 115 may be obtained at a single time point or at different time points. For example, features extracted from a Cardiac Computed Tomography Angiography (CCTA) exam performed at a baseline state, and from an angiographic exam, performed at a later time point may be used to predict the measure of interest. Similarly, blood biomarkers (the same or different) may be acquired at different time points and used as features of the ML models, as described below.

Continuing with reference to FIG. 1, at step 120, features of interest are extracted from the non-invasive patient data, the medical images, and the blood biomarkers. Then, at step 125, one or more trained ML models are used to predict / estimate measures of interest such as risk of a cardiovascular event, plaque evolution, etc. The ML models may be used in a cascaded or parallel workflow, and may be synthetic data, generated in silico. For example, the training of the ML model(s) may employ physics based models to generate the output measures of interest against which the ML model is trained. Synthetic data may be further generated to enhance the database used during the training phase and the training is performed on synthetic data. In examples not covered by the invention, ML models may furthermore be used to perform plaque histology / identification / segmentation. In one embodiment, deep learning based ML methods may be used with the ML models applied at step 125.

The measures of interest determined at step 125 can be defined at patient level or at lesion / plaque level. For example, in some embodiments the measures of interest determined at step 125 include the risk of a cardiovascular event related to atherosclerotic plaque (e.g., myocardial infarction related measures such as coronary circulation, stroke related measures such as cerebral circulation, and gangrene related measures such as peripheral circulation). The risk may be provided as a single score or as a time- dependent curve, it may refer to a specific period of time in the future, etc. In combination with the risk, an indication of a future screening date may also be provided (e.g., when to perform a follow up exam, so as to: confirm the prediction, perform an intervention if required, etc.). Additionally, plaque composition measures of interest may be predicted at step 125 such as absolute / relative values of plaque components (e.g., fibrous tissue, necrotic core, lipid pool, calcification, etc.), as well as plaque evolution-related measures of interest such as future size, shape, composition and location of plaques. Separate models may be used for the initiation, evolution, and rupture of plaque.

In some embodiments measures of interest related to in-stent restenosis may be determined at step 125. In-stent restenosis tends to appear 3-6 months after the stent placement, due to the proliferation of cells in the media layer (also called neointimal hyperplasia). Measures of interest may include, for example, degree of restenosis, time of onset, etc.

The measures of interest determined at step 125 may also identify lesions which require sealing. In combination with stents generated from bioresorbable scaffold, which have the ability to dissolve after restoring the patency of the vessel, sealing of underlying plaques prone to future growth, as predicted by ESS, may be used to improve long term evolution of coronary arterial disease (CAD) patients.

Therapy planning measures of interest may also be determined at step 125, according to an example not forming part of the claimed invention. In case a medical therapy is prescribed (e.g., following a baseline exam), the measure of interest may be related to the efficiency of the drug treatment (e.g., decrease of the risk for a cardiovascular event). In case of device therapies (such as stenting, grafting etc.), the measure of interest could be the viability/effectiveness of the device in the wake of plaque and/or thrombus initiation and growth.

Returning to FIG. 1, at step 130, the measure of interest determined at step 125 is presented to the clinician in a visualization. This visualization may be presented, for example, in a numeric (e.g., tabular) or in a graphical way (e.g., overlaid on the medical images).

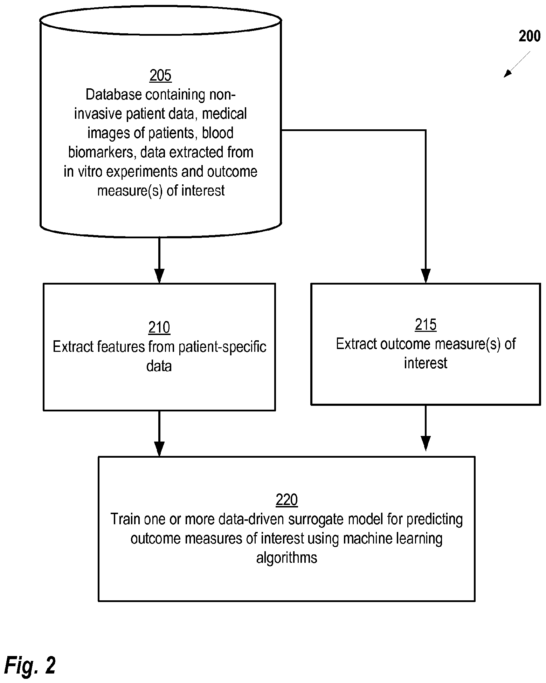

The workflow displayed in FIG. 1 is used during the prediction phase which is performed online. To be able to use the ML models, the models may be trained *a priori* offline. FIG. 2 displays the generic workflow 200 of the training phase, not forming part of the claimed invention One of the requirements for the training phase in this example is the existence of a large database 205 containing synthetically generated and/or patient-specific information (non-invasive data, medical images, blood biomarkers), corresponding ground-truth, namely the outcome measures of interest for plaque evolution (e.g., risk of a cardiovascular event, plaque evolution). The database 205 may contain information from *in vivo, in silico* and *in vitro* studies. Once this database 205 is assembled, relevant features and the corresponding outcome measure(s) of interest are extracted at steps 210 and 215, respectively. Then, at step 220, the features and outcome measures of interest are used to train one or more data-driven surrogate models using ML models. During the training phase more features may be available than during the online prediction phase. The features which are missing during the prediction phase may be estimated based on similar datasets in the training database, (e.g., by employing a separate ML model specifically trained for this purpose). In one embodiment the training of the ML model(s) employs physics based models to generate the output measures of interest against which the ML model is trained (as described in further detail below). In an example not forming part of the claimed invention, the training may not require any physics based models, and may use only features extracted directly from the input data.

One possible ML model which may be employed in workflows 100 and 200 shown in FIGs. 1 and 2, respectively, is deep tensor neural network (DTNN), which emerged from the tensor network theory applied in neuroscience (tensor interactions are encountered in the central nervous system). DTNN performs data regularization, ensuring thus that the underlying physics are captured, and leading to models which have generative power. Various variants of the DTNN may be used with the workflows 100, 200 including, without limitation, DTNNs that employ linear activations instead of sigmoidal nonlinearity may be used and directly connected to layers through a tensor, and DTNNs where the sigmoid nonlinearity is applied after the Kronecker product, instead of being applied directly to the double projection layers.

In some embodiments, the ML models utilized herein employ deep learning methods. The term "deep learning" generally refers to a category of artificial intelligence techniques, which are based on a plurality of information-processing layers. Hierarchical structures are employed, either for the learning features or representation or for classification / regression. Recently, deep learning based applications have improved on prior state-of-the-art results in several areas of research, like voice recognition (natural language processing), computer vision, optical character recognition, signal processing, information retrieval, etc.. The deep learning techniques may be categorized into synthesis / generation or recognition / classification. Thus, three main deep learning classes may be considered. Within the first class, discriminative models are developed for delivering high discriminative power either in classification or regression applications. In the second class, generative models are developed for characterizing the higher-order correlation characteristics of the available data for synthesis / analysis. These models may be employed to characterize the mutual statistical properties of the data and the corresponding classes. If, additionally, a Bayes rules is used, a discriminative version of the model is generated. Finally, in the third class, hybrid models may be utilized to perform both discrimination (classification / regression) and characterization of the samples.

One example of a generative model is a deep autoencoder which is a special type of deep neural network that outputs a transformed version of the input data. It is used for learning either dimensionality reduction or efficient encoding. It extracts non-linear features without making use of class labels. Thus, an autoencoder is generative and employs at least three layers: an input layer comprising input data; an output layer that same size as the input layer, whereas each unit corresponds to exactly one unit in the input layer; and one or more hidden layers (typically with fewer units than the input and output layers) that generate the encoding.

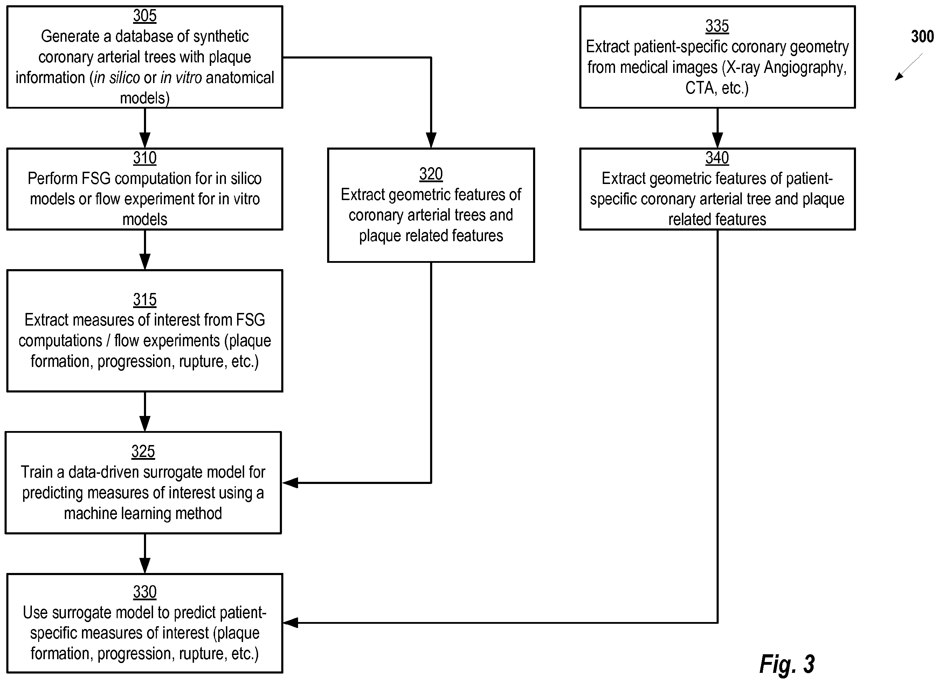

To further illustrate the techniques described herein, an ML model is trained based on a synthetic data to predict patient-specific plaque related measures of interest (e.g., plaque formation, progression, rupture, etc.). Here, synthetic data refers to data that is not specific to a particular individual, but rather data generated by using an algorithm, model, or a physical experiment. FIG. 3 illustrates a workflow 300 for computing patient-specific plaque related measures of interest for this example. Starting at step 305, a database is generated with synthetic coronary arterial trees. These trees may be generated using either *in silico* or *in vitro* techniques, wherein only the in silico techniques are covered by the claimed invention. Next, at step 310, fluid solid growth (FSG) computations are performed for the in silico anatomical models or flow experiments for the in vitro anatomical models. Various types of plaque growth models (described above) may be applied ranging from very simple to very complex models. These plaque growth models can be coupled with the fluid-solid interaction model. Based on these computations/experiments, one or more plaque related measures of interest are extracted at step 315. In parallel with steps 310 - 315, geometric and/or morphological features are extracted from the anatomical model at step 320. Then, at step 325, data-driven surrogate model(s) are trained using the geometric features and the target measure(s).

Continuing with reference to FIG. 3, once the surrogate model has been trained, it is applied at step 330 to predict patient-specific geometries obtained from medical images (X-ray angiography, CTA, etc.). For example, at step 335 in FIG. 3, patient-specific coronary geometries are extracted from medical images. The geometric features of the patient-specific coronary arterial tree and plaque-related features are next determined at step 340. These features may then be used as input data for the surrogate model. The missing features can be either predicted from a separate ML model, or estimated using similar anatomies in the database of synthetic geometries. FIG. 3 refers specifically to the coronary circulation, but the methodology may be applied similarly for other parts of the cardiovascular system.

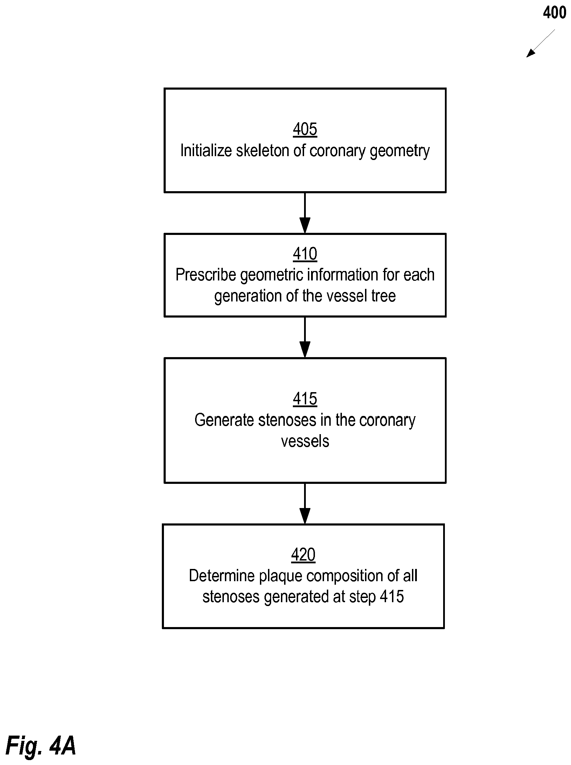

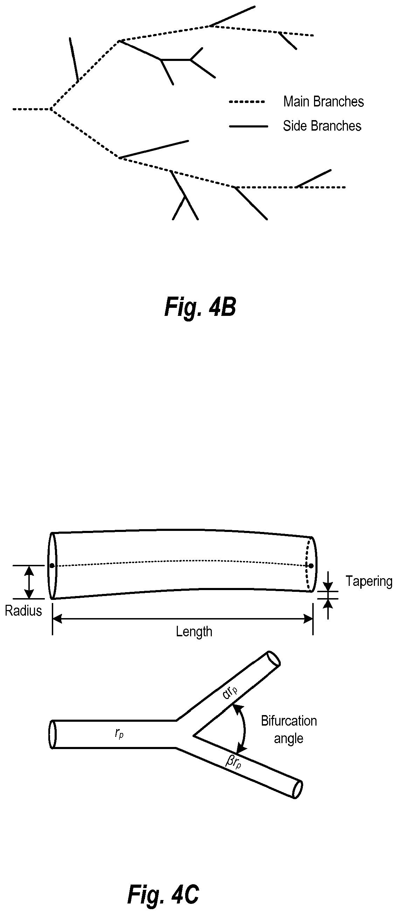

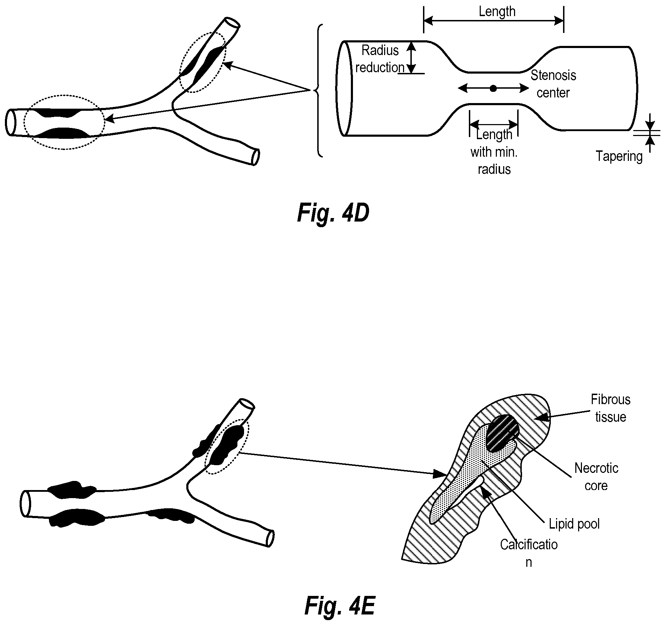

FIG. 4A illustrates a method 400 for generating synthetic *in silico* data, according to some embodiments. Starting at step 405, the skeleton of the coronary geometry is initialized, by prescribing the number of vessels at each generation of the tree (as visualized in FIG. 4B). At step 410, geometric information such as vessel radius, degree of tapering, branch length is prescribed for each generation of the vessel tree (as visualized in FIG. 4C). At step 415, stenoses are generated in the coronary vessel trees (as visualized in FIG. 4D). Then, at step 420, the plaque composition for all stenoses generated at step 415 is determined (as visualized in FIG. 4E).

Aside from the plaque composition determined at step 420 of the method 400, in some embodiments, other plaques may be generated which do not cause a constriction (e.g., plaques which lead only to positive remodeling). Various approaches can be followed to determine the type / composition of these synthetically generated plaques. For example, in some embodiments, the type of the synthetically generated plaques is randomly selected from a predefined list of plaque types (e.g., thin-cap fibroatheroma, predominantly fibrous plaque, etc.). Random selection may also be used to select the center of each volume of plaque material (e.g., center of necrotic core, center of lipid core, etc.), as well as the shape and size of each volume of plaque material and its material properties. Additionally, the synthetically generated plaques may be generated so as to mimic a geometric appearance that is typically associated with "high risk plaque", such as a napkin-ring shape and spotty calcification, a thin cap, etc. An important aspect is also the determination of the material properties of the arterial wall; these are prescribed during step 410.

Some of the flow related features that may be used by the ML models have been previously described in United States Patent No. 9,349,178 to Itu et al., issued May 24, 2016, entitled "Synthetic data-driven hemodynamic determination in medical imaging. Additionally, plaque related features may be extracted including, without limitation, the size/shape/volume of the plaque, the location of the plaque, the degree of positive remodeling, and the plaque composition (e.g., volume / percentage for each type plaque component). Furthermore, more advanced features can be defined, similar to the ischemic weight and ischemic contribution score. These advanced features may include, without limitation, plaque formation score (e.g., features describing the likelihood of plaque development at a certain location) and plaque growth score (e.g., features describing the growth speed of the plaque. Different growth factors can be defined for the different components of the plaque. Additionally, advanced features may be defined based on plaque rupture score (e.g., features describing the likelihood of a rupture of the plaque and plaque thrombus score (e.g., features describing the likelihood of thrombus formation of the plaque surface). The aforementioned advanced features may be derived from the basic features (e.g., geometry, plaque morphology, etc.) using mathematical operators, or may be determined by separate ML models.

The above described methodology refers to a workflow where the synthetic coronary geometries are generated directly with plaque information. Alternatively, healthy coronary geometries could be generated (with no plaque), and the ML model could predict the locations of plaque formation. The feature set could, in this case, be augmented with other synthetically generated features that are extracted from the patient data during the online application of the learned model. These features could be related to blood biomarkers (e.g., cholesterol), characteristics like arterial systemic pressure, or demographics.

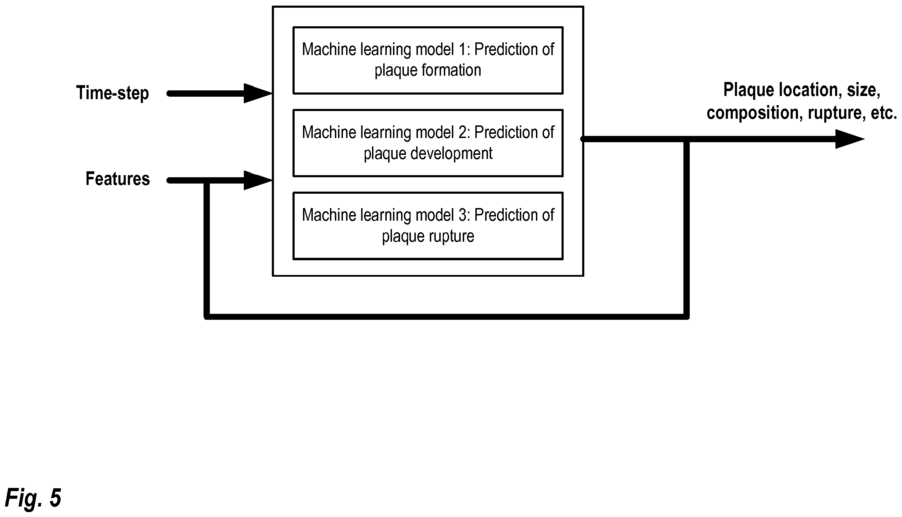

In some embodiments, multiple types of ML models could be trained based on different FSG model versions. As illustrated in FIG. 5, these models can then be used in parallel for the prediction of plaque formation, development, and rupture. Each model uses as input a set of features (as described above), and also a time-step, to determine the various plaque related measures of interest for a certain future moment in time. This parallel model approach can then be applied iteratively to determine the evolution in time of the plaques / patient.

Additionally, a confidence metric could be associated with the outputs. For example, this confidence metric can be defined such that the smaller the time step / the shorter the time-span predicted by the ML model, the higher the confidence will be.

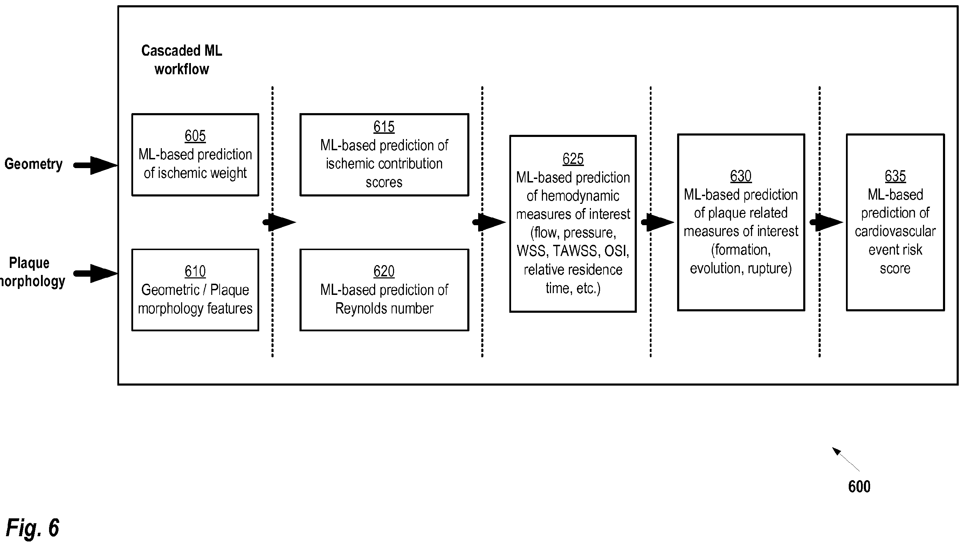

FIG. 6 illustrates a cascaded ML-based workflow 600 for the prediction of plaque related measures of interest, according to some embodiments. As mentioned above, the more advanced features could be themselves generated with ML models, leading to a multi-level cascaded approach. The example of FIG. 6 uses as input only geometrical features of the vessel anatomy and of the plaque morphology. Each level in this cascaded workflow uses as features the outputs of the preceding ML levels. At the first level, the ischemic weight of each branch is computed at step 605 and the geometric / plaque morphology features are determined at step 610. At the second ML level, the ischemic contribution scores are computed at step 615 and the Reynolds number at each location of the arterial structure is determined at step 620. Next, at the third ML level hemodynamic measures of interest (e.g., flow, pressure, WSS, TAWSS, WSSG, OSI, relative residence time, presence of helical flow, tissue stress, etc.) are predicted at step 625. During the fourth ML level, the plaque related measures of interest are computed. Finally, at the fifth ML level the risk of future cardiovascular event is predicted at step 630.

The ML models described above use completely synthetic data during the training phase. The result predicted by the ML model for patient-specific data can potentially be improved by using patient characteristics. Hence, in the above described cascaded ML workflow, an additional level may be introduced. First a result is predicted by the algorithm which was taught on purely synthetic data and, then, the final ML algorithm uses the result predicted by the previous algorithms as features, alongside patient characteristics, in order to improve the final prediction.

The features for the second ML algorithm may include, for example, patient demographics, pathological history, previous cardiovascular history, non-invasive stress test results, exercise electrocardiogram (ECG) stress test results, exercise radioscope test results, blood biomarkers, and medications used in the past or present by the patient. One possibility is to build a database with the patient-specific data of previous cases and to use this database during the cascaded ML approach. As described before, during the first step, the ML algorithms taught on synthetic data are used to generate a first prediction of the measure of interest. During the second step, the features extracted for the patient specific data are used to find similar cases in the patient database and a final ML model is applied for predicting the final value of the measure of interest.

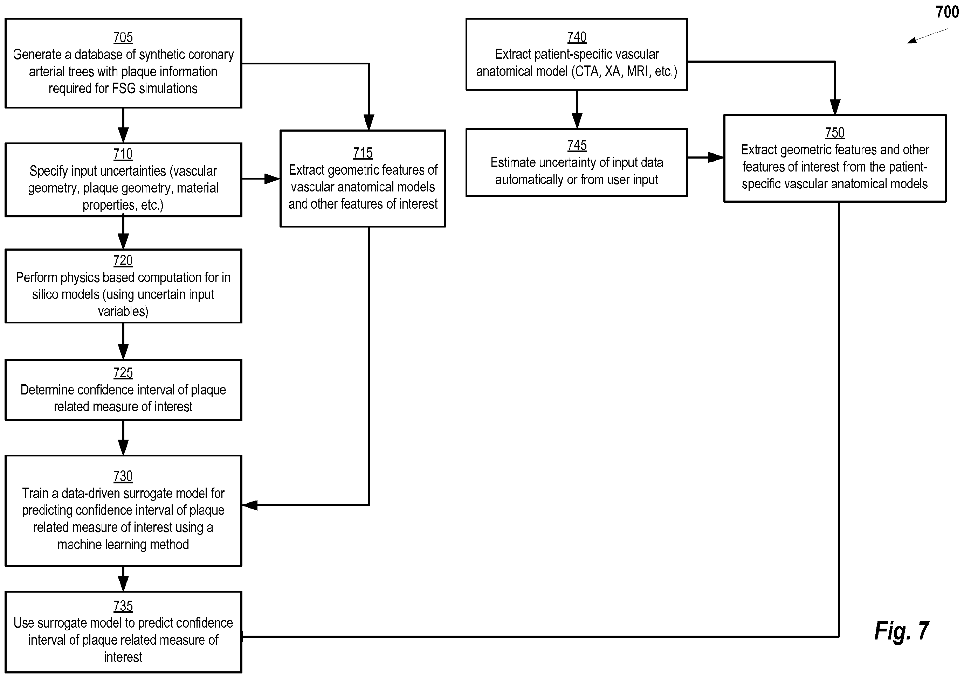

In some embodiments, separate ML models may be used to provide a confidence interval for the estimation of a plaque related measure of interest, as shown in the workflow 700 presented in FIG. 7. Briefly, the first step is to use a set of uncertain input variables in the synthetic data (e.g. vessel diameter, plaque volume, etc.), propagate this uncertainty through the FSG model and determine the uncertainty for the measure of interest. The uncertainty can then be learned through an ML model based on the extracted features. The same features are then extracted for a patient-specific geometry and uncertainty in the input data is specified either automatically or by the user, and, using the ML model, the confidence of the estimated measure of interest metric is provided. Because such a ML model requires a large database, FIG. 7 is described below with reference to a database composed of synthetic data; however, it should be understood that a database comprising synthetic and / or patient-specific data may likewise be used. Additionally, FIG. 7 refers specifically to the coronary circulation, but the methodology detailed in this example may be applied similarly for other parts of the cardiovascular system.

Starting at step 705 in FIG. 7, a database is generated comprising synthetic coronary trees with plaque information, as required for FSG simulations. Next, at step 710, input uncertainties are specified, for example, by the user or based on previously used settings. These input uncertainties may pertain to, for example, the vascular geometry, the plaque geometry, material properties, etc. Based on the information collected at steps 705 and 710, at step 715, the geometric features of vascular anatomical models and other features of interest are extracted. Next, at step 720 physics-based computations are performed for *in silico* models using the uncertain input variables specified at step 710. Then, based on the results generated from step 720, a confidence interval is determined at step 725 for each plaque-related measure of interest.

Continuing with reference to FIG. 7, at step 730, a data-driven surrogate model is trained to predict a confidence interval of plaque related measures of interest using an ML-based method. At steps 740 - 745 a patient-specific vascular anatomical model is extracted from imaging data (CTA, XA, MRI, etc.) and the uncertainty of the data is estimated automatically or from user input. Additionally, at step 750, geometric features and other features of interest are extracted from the patient-specific vascular anatomical models. Finally, at step 735, the surrogate model is used to predict a confidence interval of the plaque-related measure of interest using the data generated at step 740 - 750.

As described above, one possible application of the techniques described herein is to determine a future screening date for the patient. Thus, when the patient undergoes this screening exam, a new dataset, besides the baseline data becomes available. In general, if multiple datasets are available for a patient, this additional information can be used to further improve the ML based predictions. For example, the features extracted from the additional information may be used directly as input features. Also, the features extracted from the additional information may be used at an intermediate level of a cascaded ML approach (e.g., for the workflow in FIG. 6). These additional features may be used as input data at the fourth level to obtain a better prediction of plaque formation, evolution and rupture. Furthermore, subsequent exams may be scheduled not only for screening purposes, but also in case of minor / major cardiovascular events.

In some embodiments, the techniques described herein are capable of including information extracted from measurements: if the measurement of a plaque related feature is available (e.g., as acquired through IVUS or OCT), the system can use this information to improve the accuracy of the prediction everywhere. Further, the error in the original prediction at the location where data is provided can be used to improve the model's future performance. The users' corrective actions taken to improve automatically identified features can be used to improve the feature detection in the future. The system may be able to learn from the user inputs. Further, if additional data on the outcomes becomes available, the learned model can itself be continuously improved. This improvement can be performed at global level or in a site-specific manner. This allows the system to account for anatomical trends based on patient demographics. If measurements of plaque related features become available, the system can automatically or semi-automatically identify outlier cases. These cases can then be used to create a new set of synthetic geometries which mimic the features of the outlier, together with the already available training set to improve the model predictions. In addition to anatomy, if plaque related measurements are also available (e.g., via IVUS or OCT), then they can also be incorporated in the machine learning approach. To do this, the training data is appended with new features characterizing plaques. In the prediction phase, if the measured values of these 'plaque' related features are available, they could be used as features. In their absence, the model can be used to find similar patients from the database from the geometric features to arrive at data-driven estimates of plaque features in different branches.

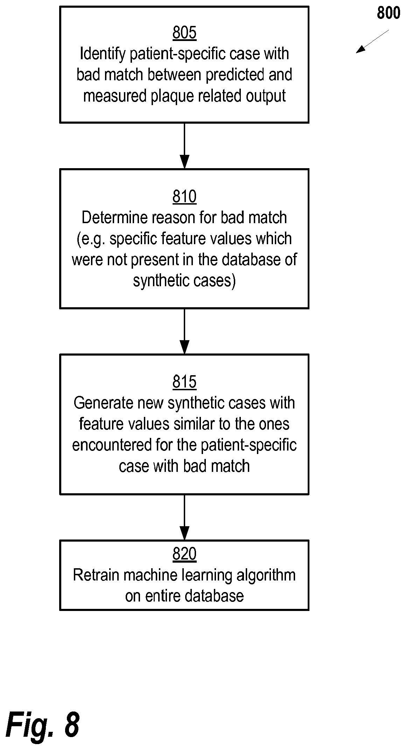

Although a very large number of synthetic cases can be generated for training the ML algorithm, these will typically not cover all patient-specific cases. Hence, when using the ML algorithm to predict results for patient-specific data, bad matches between predicted and measured output might appear. In this case, the workflow 800 displayed in FIG. 8 can be used to enrich the database of synthetic cases so as to improve the prediction for the patient-specific cases which lead to a bad match. The workflow 800 may also be performed directly on the workstation (since the generation of synthetic data can be fully automated). Starting at step 805, a patient-specific case that is a bad match between the predicted measurements and the acquired patient-specific measurements is identified, for example, manually by the user or automatically with an ML-approach based on the presence of one or more features in the data. The term "match" here is somewhat flexible in that the tolerance for determining when two measurements are equivalent can be adjusted as desired. For example, in some embodiments, the acceptable range of values for the predicted measurements may be limited to values within 1% of the measured values. In other embodiments smaller or larger percentages may be used depending, for example, on the type of data being measured. Continuing with the workflow 800, at step 810, the reason for the bad match is determined, again either based on user input or using an ML model. At step 815, new synthetic cases are generated with feature values similar to the ones encountered in the case with the bad match. Then, at step 820, the ML model is retrained on the entire database.

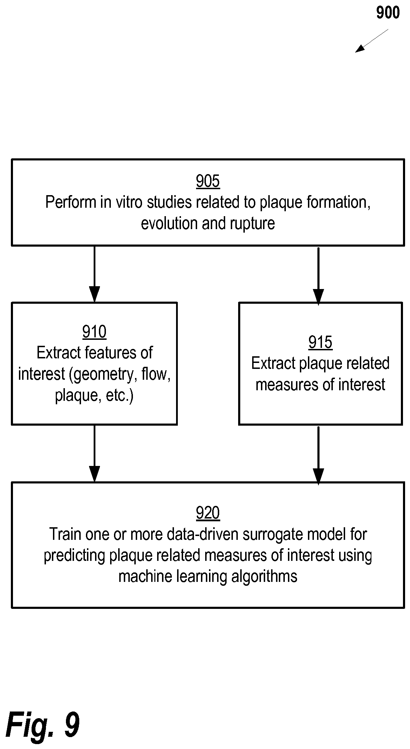

*In vitro* studies are an important alternative for studying plaque formation, evolution and rupture. The following examples related to in vitro studies and models do not fall within the scope of the claims and are to be interpreted as examples useful for understanding various embodiments of the invention. Since the experimental conditions can be controlled exactly in in vitro studies, an ML model can be trained to predict the formation, evolution and rupture of plaques using a database built from such studies as shown in the workflow 900 presented in FIG. 9. At step 905, *in vitro* studies are performed related to plaque formation, evolution, and rupture. Next, at steps 910 and 915 features of interest and plaque-related measures of interest are extracted. The features of interest may include, without limitation, the geometry of the artificial vessel, the hemodynamics in the artificial vessel (e.g., flow rate, velocity, pressure, shear stress, etc.) obtained through direct or indirect measurements, and plaque composition. Then, at step 920, one or more data-drive surrogate models are trained for predicting plaque-related measures of interest using ML models.

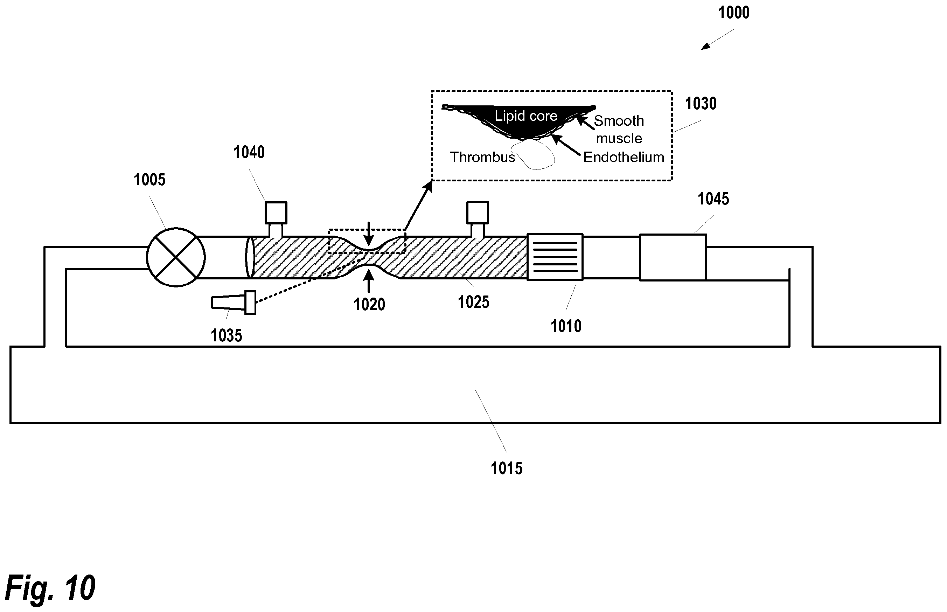

FIG. 10 illustrates an example *in vitro* model 1000 that may be used for generating the training database, said model not being covered by the claimed invention. The model 1000 includes an Artificial *In Vitro* Vessel 1025 (or vessel tree). A Pump 1005 circulates a fluid with properties similar to the ones of human blood through the in vitro model. Hydraulic Resistances 1010 are coupled to the terminal in vitro segments. Together with the Pump 1005, the Hydraulic Resistances 1010 generate realistic levels of pressure inside the in vitro model 1000. A Reservoir 1015 collects the fluid. One or more Occluders 1020 generate constrictions in the in vitro model 1000.

Cardiovascular Tissue 1030 (endothelium, plaque material, etc.) is placed on the inside surface of the Artificial *In Vitro* Vessel 1025. For example, basic human in vitro models of atherosclerosis may comprise simple endothelial cultures treated with certain risk factors (inflammatory factors, oxidized low density lipoprotein, high levels of glucose) and co-cultures which include human leukocytes or smooth muscle cells. The model 1000 may also include various measurement devices for determining the hemodynamic measures including Pressure Transducers 1040, Flow Meters 1045, and a Doppler Probe 1035 for measuring the fluid velocity. Additionally, a pressure or flow measurement catheter (not shown in FIG. 1000) may be used to add the influence in high degree stenosis or small diameter vessels on the measured quantity.

The in vitro model 1000 may be modified in numerous ways to generate a large number of setups. For example, the following characteristics may be varied in different embodiments: the number, position and shape of the occluders; resistances; pump action; number of side branches (total occlusion may be induced); heart movement, breathing (4d in-vitro model); heart rates, flow, flow curves, any specific coronary or heart disease, bypass, effect of collateral flow; plaque type (e.g., TCFA, eroded plaque, etc.); plaque composition; fluid composition (e.g., cholesterol level). Furthermore, the above described in vitro studies may be performed at normal or microfluidic scale, and in 2D or in 3D.

*In vitro* studies, similar to the one displayed in FIG. 10, may be alternatively used to derive a simplified version of a mathematical representation of plaque formation, evolution and rupture (simplified version of plaque related governing equations). Although great advances have been reported in developing ML-based models for real-time prediction of dynamic processes, these models can only be applied under conditions which were considered during the training of the ML model. Hence, these models cannot extrapolate beyond the range of feature values considered during the training phase.

To extrapolate beyond the range of feature values considered during the training phase, in some examples not covered by the invention an approach based on the theory of dynamical system discovery is utilized. The main idea is that the majority of physical processes can be modeled with only a few terms that are most relevant for the dynamics of the system. Thus, the governing equations can be considered to be sparse in a high-dimensional nonlinear function space, and represented as:

$$\frac{d}{dt}\boldsymbol{x}(t) = \boldsymbol{f}\big(\boldsymbol{x}(t)\big),$$

where x is the state vector and f represents the dynamic constraints of the system. Note that, with this approach, the time history of the state vector is collected and used. Model complexity and accuracy are naturally balanced. The derivative of the state vector is either measured or determined numerically from the time history of the state vector.

An important step of the identification problem is the specification of the candidate nonlinear functions (there is no limitation in the choice of functions). If basic knowledge of the physics behind a given identification problem is available, it may be used to derive candidate nonlinear functions. For example, in case of plaque related applications, various growth models have been proposed in the past. Various approaches may be then used to determine which nonlinear terms are active for a given identification problem.

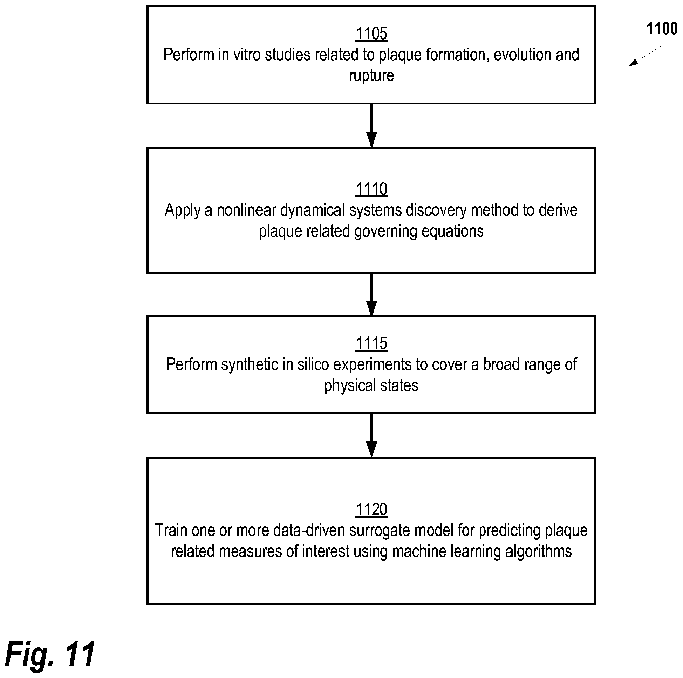

FIG. 11 shows a workflow 1100 for training an ML model on *in silico* data generated from a physics based model derived from *in vitro* data, not forming part of the claimed invention. This workflow 1100 is an extension of the workflow in FIG. 9, which is based on the above described methodology for dynamical system discovery. Starting at step 1105, *in vitro* studies are performed which span a large but finite variety of conditions (in terms of geometry, hemodynamics, plaque composition and structure, etc.). At step 1110, the nonlinear dynamical systems method is applied to derive simplified governing equations related to plaque formation, evolution and rupture. Next, at step 1115 a methodology similar to the one described above with respect to FIG. 4 is used for generating synthetic *in silico* data, herein based on the models derived at step 1110. Then, at step 1120, one or more ML models are trained for predicting in real-time plaque related measures of interest based on the synthetic data generated at step 1115.

This workflow 1100 shown in FIG. 11 combines the advantages of the methods and workflows described in the previous sections, because it incorporates information extracted from *in vitro* models simulating realistic plaque related hemodynamics. Because simplified governing equations are learned based on these experiments, one can extrapolate beyond the conditions encountered in the *in vitro* studies (due to objective constraints like time and budget, typically only a limited amount of *in vitro* studies can be performed). However, depending on the nature of the derived governing equations, these may allow only for numerical solutions, which in turn require large execution times. By running synthetic *in silico* experiments based on the governing equations, and learning the mapping between a given set of input conditions and the plaque related measures of interest, real-time based prediction can be achieved.

An alternative to the nonlinear dynamical systems discovery method has been recently introduced and termed field inversion and machine learning (FIML). This technique has been used for complementing existing mathematical models so as to obtain a better match with experiments. Inverse modeling techniques are employed to derive corrective terms (which may be spatially and / or temporally distributed), and ML techniques are used to reconstruct the model corrections (i.e., additional functional forms that appear in the model).

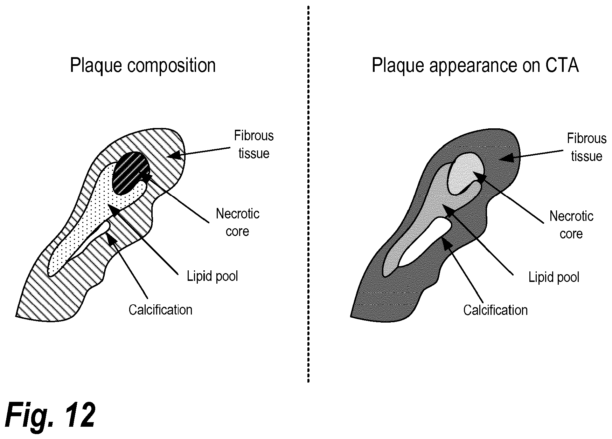

ML models may also be used to perform plaque histology on medical images. In an example not covered by the invention, FIG. 12 shows on the left an atherosclerotic plaque with four main components and on the right side its appearance on a CTA image: different plaque constituents have different densities and thus lead to a different appearance on medical images (e.g. lipid density was found to be lower than collagen or smooth-muscle density).

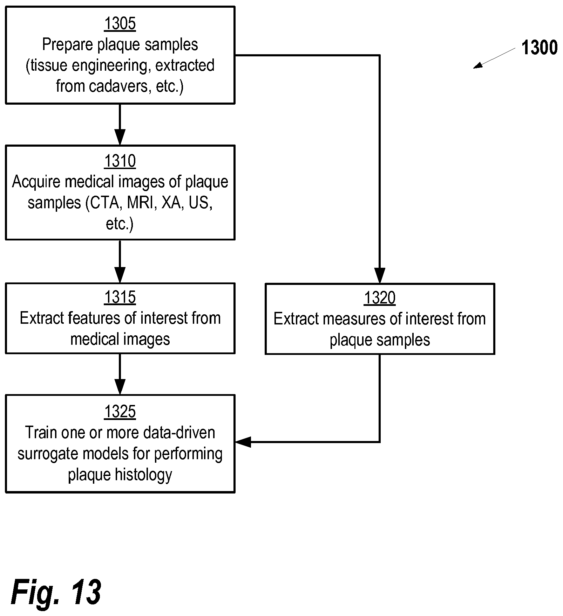

FIG. 13 shows a workflow 1300 for training an ML model to perform image-based plaque histology, not forming part of the claimed invention. At step 1305, plaque samples are prepared. These may be obtained either through tissue engineering, cadaver extractions, endarterectomy (e.g. carotid), etc. At step 1310, medical images of the plaque samples are acquired using any available medical imaging technologies (CTA, MRI, XA, US, etc.). At step 1320, the measures of interest are extracted based on the plaque samples (i.e. plaque histology is performed). At step 1315, plaque related features of interest are extracted from the acquired medical images. These features may include, without limitation, grayscale values in different parts of the image, grayscale values of neighboring regions, etc. Furthermore, in some illustrative examples not being covered by the claimed invention, arterial wall strain may be used as a feature related to plaque composition (low strain - fibrous tissue; large strain - lipid tissue). Then, at step 1325, one or more ML models are trained to perform plaque histology based on the medical images.

Such ML models would be able to compensate for example for the blooming artifacts present on a CTA image in case of calcified plaque. Radiogenomics features may also be employed in the ML workflow (imaging biomarkers that are linked with the genomics of a pathology). In a different illustrative example, plaque related data acquired through intravascular medical imaging technologies (IVUS, OCT, etc.) may be used for determining the ground truth values of the measures of interest. For example, a set of patients may be investigated both through CTA and IVUS. IVUS-based virtual histology may be used to determine the main constituents of each plaque and then used to train the aforementioned ML models.

In a different example not forming part of the claimed invention, a modified version of the workflow 1100 displayed in FIG. 11 may be employed to perform image-based plaque histology. For example, a nonlinear dynamical systems discovery method may be used to derive the governing equations of medical image generation (using either in vitro studies - real tissue samples, or virtual histology studies). Then, derived governing equations may then be used to generate a large number of pairs of synthetic plaque compositions and corresponding synthetic images. Finally, the ML models discussed with respect to FIG. 11 can be trained for performing image based plaque histology.

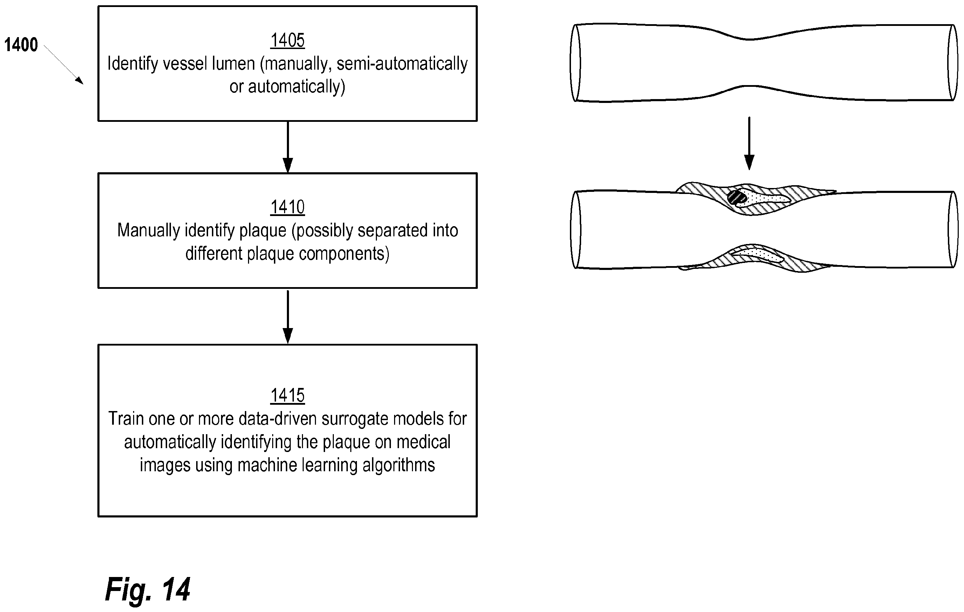

In another example not forming part of the claimed invention, ML models may be used to identify plaques on the medical images (and possibly also the plaque composition). For example, FIG. 14 displays on the left a workflow 1400 for training one or more ML models for identifying plaques on medical images. Starting from a large database with medical images of vessels, at step 1405, vessel lumen is identified on images. This may be performed automatically, semi-automatically or fully automatically. Next, at step 1410, plaque is manually identified (segmented) on the images. The plaques may be associated with vessel narrowings (as displayed in the right hand portion of FIG. 14) or not. Step 1410 may involve, for example, drawing of contours, identifying a set of points on the boundary of the plaque (of the different plaque constituents), etc. Then, at step 1415, one or more ML models are trained to identify the plaques on medical images. The first two steps may also be performed simultaneously in some embodiments. Advanced learning techniques like reinforcement learning may be combined with deep learning techniques to specifically learn based from the actions performed by the user while manually identifying / segmenting the plaque.

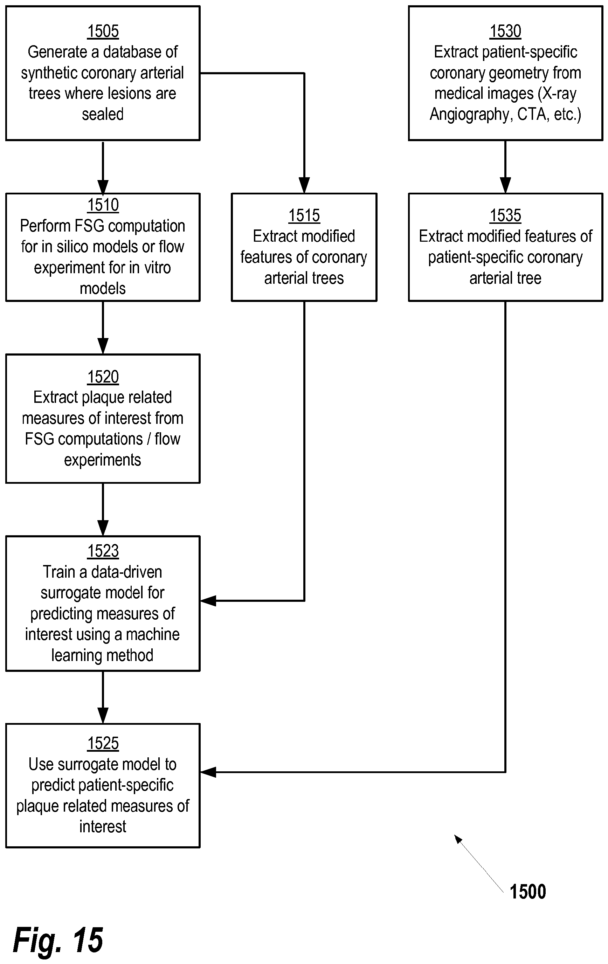

According to an illustrative example not forming part of the claimed invention, an ML algorithm may also be used to identify vulnerable lesions (which may or may not be flow-limiting). FIG. 15 illustrates a workflow 1500 for performing individual lesion assessment. In this workflow 1500, the vulnerable lesions are automatically identified and virtually sealed. The algorithm used for assessing the plaque related measures of interest is adapted so as to remove the effect of the vulnerable lesions on the hemodynamics and the future evolution of the anatomical model (in this case the initial geometry does not have to be modified).

Starting at step 1505 of FIG. 15, a database of synthetic coronary arterial trees where lesions are sealed is generated. Next, at step 1510, FSG computation is performed for *in silico* models or flow experiment for in vitro models. At steps 1515 and 1520, respectively, modified features of coronary arterial trees are extracted from the database and plaque related measures of interest are extracted from FSG computations / flow experiments. Then, at step 1523, a data-driven surrogate model is trained for predicting measures of interest using a machine learning method.

Continuing with reference to FIG. 15, at step 1530, a patient-specific coronary geometry is extracted from medical images (X-ray Angiography, CTA, etc.). Next, at step 1535 modified features of a patient-specific coronary arterial tree are extracted. For example, one approach would comprise modifying the features related to the growth of the plaque. Then, at step 1525 the surrogate model is used to predict patient-specific plaque related measures of interest. Similar algorithms / workflows may be used in case of device therapies (such as stenting, grafting etc.), whereas the measure of interest could be the viability / effectiveness of the device in the wake of plaque and / or thrombus initiation and growth.

Another important application in the context of atherosclerotic plaque not being covered by the claimed invention, is drug-based therapy planning. The typically prescribed drugs for treating atherosclerosis include statins (decrease the level of LDL cholesterol); fibrates (decrease the level of triglyceride); nicotinic acid (reduce both triglycerides and LDL); ezetimibe (decrease the level of cholesterol absorption in the digestive system; bile acid sequestrants (decreases bile acid level, which in turn increases cholesterol usage, leading to lower cholesterol level); and anti-inflammatory interleukin (IL)-13 (induce plaque stability).

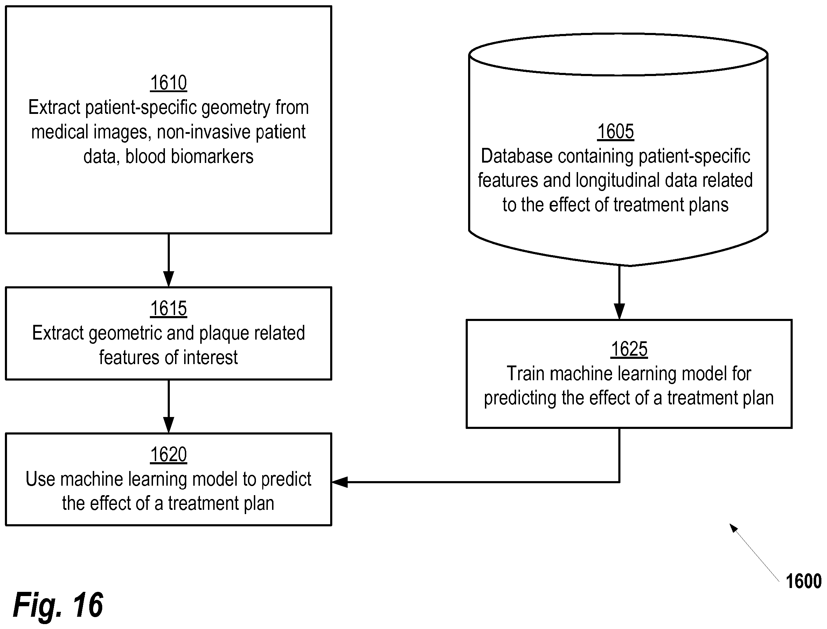

According to an example not covered by the claimed invention, an ML based workflow may be used to estimate the effect of different drug based treatment plans and chose the best possible treatment plan for each patient as shown in the workflow 1600 presented in FIG. 16. A key element for this workflow 1600 is the existence of a database 1605 containing longitudinal data related to different treatment plans for various patients. This may be generated, for example, based on data acquired at different time points. This database is used at step 1625 to train an ML model to predict the effect of a treatment plan.

Continuing with reference to FIG. 16, at step 1610, patient-specific geometry is extracted from medical images (XA, CT, MRI, etc.), non-invasive patient data (demographics, patient history, etc.), and blood biomarkers. Next, at step 1615, geometric and plaque related features of interest are extracted from the patient-specific geometry. Finally, at step 1620, the trained ML model is used to predict the effect of a treatment plan based on the geometric and plaque related features of interest.

Separate ML models may be trained for different drugs (statins, fibrates, niacin, ezetimibe, bile acid sequestrants); different combinations of drugs ; assessing the effect of changes in treatment plans; patients which have already suffered cardiovascular events; and patients without cardiovascular events The outcome measures of interest predicted by the ML model may be an optimal treatment plan (e.g., which drug, which quantity, etc.), lowering plaque growth rate / stopping plaque growth, decrease of cardiovascular event risk, etc. Additionally, a cascaded ML approach may be used in some illustrative examples not forming part of the claimed invention (e.g., as described above with reference to FIG. 6). The first ML model in the cascade may focus on the prediction of hemodynamic and plaque related measures of interest and the second ML model may focus on the prediction of the effect of the treatment plan.

Computed results can be visualized on the scanner, or on another device, such as an imaging workstation. All of the above mentioned measures of interest related to plaque may be visualized including, without limitation, risk of a cardiovascular event related to atherosclerotic plaque (single score or as a time-dependent curve); future screening date; plaque composition (e.g., absolute / relative values of plaque components in a plaque); plaque evolution related measures of interest (e.g., future size, shape, composition and location of plaques); formation, evolution, and rupture of plaque; instent restenosis related measures of interest (e.g., degree of restenosis, time of onset, etc.); lesions which require sealing; and therapy planning (e.g. effectiveness of a drug treatment, viability/effectiveness of a device in the wake of plaque and/or thrombus initiation and growth).

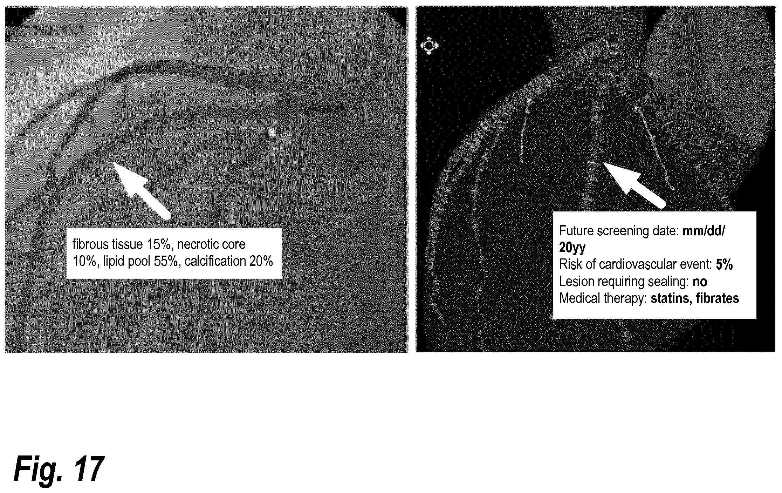

An example based on a coronary CTA image is displayed in FIG. 17. Any point on the image (related or not to a coronary plaque) can be queried (point & click) for the associated value of the measure of interest, and the corresponding value is shown overlaid to the image. As an example, points of interest / plaques can be selected and the corresponding plaque composition, future screening data, risk of cardiovascular event associated with the plaque, etc. can be displayed (FIG. 17). Alternatively, in some embodiments, the user can activate a "no click" mode, in which case the value of interest is displayed in correspondence of the cursor by just positioning the cursor on the position of interest. The system may provide a touch screen enabling interactions with the anatomical object of interest (e.g., gestures to rotate, zoom, pan, etc.). Point and touch functionality may cause the system to display the value of interest at the point of touch. Furthermore, the anatomical model of the vessels may be color-coded based on the values of the measure of interest (a continuous or a discrete color map may be used for this purpose). The above mentioned approaches may be used for a 3D or 2D visualization, which may be based on projections of the results on a plane.

When different modalities or multiple acquisitions from the same modality (one for feature extraction, another one for visualization) are used, registration of the images and features (spatial and temporal) is an important prerequisite. This can be done, for example, by specifying information of the image systems (in case they are registered), running algorithms on the images, or manually by the user by selecting land marks. When the same scanner is employed during examination and visualization, features can be table position, angulations, etc. If the measure of interest is a value representative for the entire anatomical model (e.g., risk of cardiovascular event / future screening date), this value may be displayed on a screen. The user may consider different treatment options, and the different outcomes may be displayed simultaneously on the screen / image so as to enable a fast evaluation of the different options. The visualization of plaque related measures of interest may be combined with the visualization of lesion specific diagnostic indices (e.g., coronary FFR). This may further inform the user with respect to which lesion should be treated.

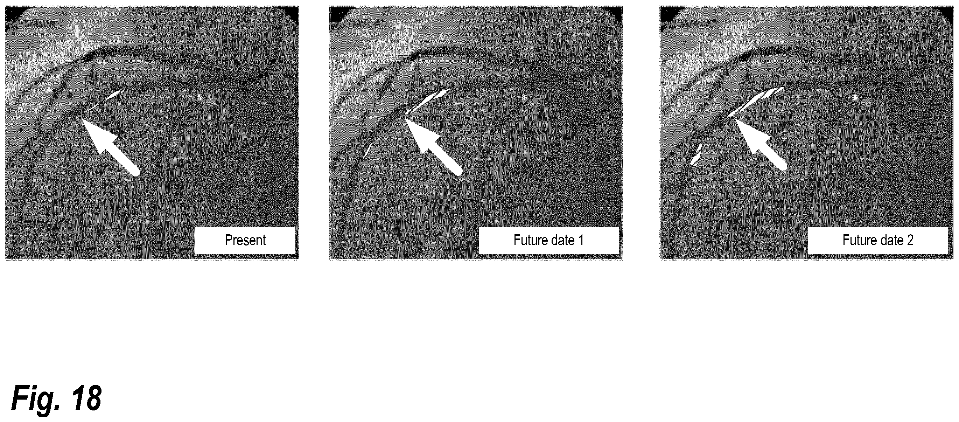

Another example is displayed in FIG. 18. Different images are generated for different future dates, whereas the predicted plaques and plaque sizes are displayed: the plaque size increases progressively, and also new plaques form.

Various additional features, enhancements, and other modification can be made to the learning-based generative methods described herein to provide additional extensions to the disclosed techniques. In an illustrative example not forming part of the claimed invention, the learning-based generative methods may be used to also predict the formation of aneurysms, dissections, inflammation or other such disease of the vascular wall. In other illustrative examples, the methods may be used to also predict the formation of myocardial scar (due to insufficient oxygen supply), onset of angiogenesis, or the formation of tumors in various organs. Alternatively (or additionally), the learning-based generative methods may be made available as a service, which may be called by a hospital / clinician to obtain a patient-specific prediction related to the aspects mentioned above.

One advantage of ML models is that the online prediction is extremely fast; it outputs results almost instantaneously (in a matter of seconds). For example, they can be run directly on the workstation located at clinics/hospitals. However, there may be situations in which a hybrid on-site - off-site processing workflow may be required. For example, off-site processing can provide more detailed information or additional information that would not be available on-site, which is enabled by the less strict requirement on the processing time. Examples of such scenarios include employing a complex computational model available off-site but not on-site, providing different analyses or options as compared to the on-site processing (e.g., therapy planning may only be available off-site), etc. Similarly, on-site assessment may not be available at the time when the medical images are acquired. This may be due for instance to limitations of the imaging workstation (incompatible hardware or software configuration), or unavailability of the workstation providing the processing functionality. In this case, off-site processing can be offered as an alternative, to produce the same results as the on-site counterpart, or with the possibility of choosing different analyses or options.

Moreover, the on-site assessment can be inconclusive or uncertain due to intrinsic uncertainty of the quantity of interest. In this case, off-site processing can include consulting medical experts (human or databases) to find the best course of action, for instance based on previous clinical cases with similar characteristics. In another scenario, the on-site assessment provides a first approximation of the quantity of interest (for instance, not all image features can be extracted with confidence); in this case, off-site processing can include further image processing to extract more image features or with more confidence / less uncertainty. Off-site processing can also include evaluating a larger set of features (e.g., nonimage features such as clinical history of the patient, risk factors for fractures, etc.) that can be incorporated in the predictor to improve the assessment. If the same type of data is available at different time points, the data acquired at the current time point may be analyzed on-site and then sent off-site for a comparative analysis with the previous acquisition. This may be used to determine the evolution of the pathology / patient so as to propose the optimal treatment strategy.

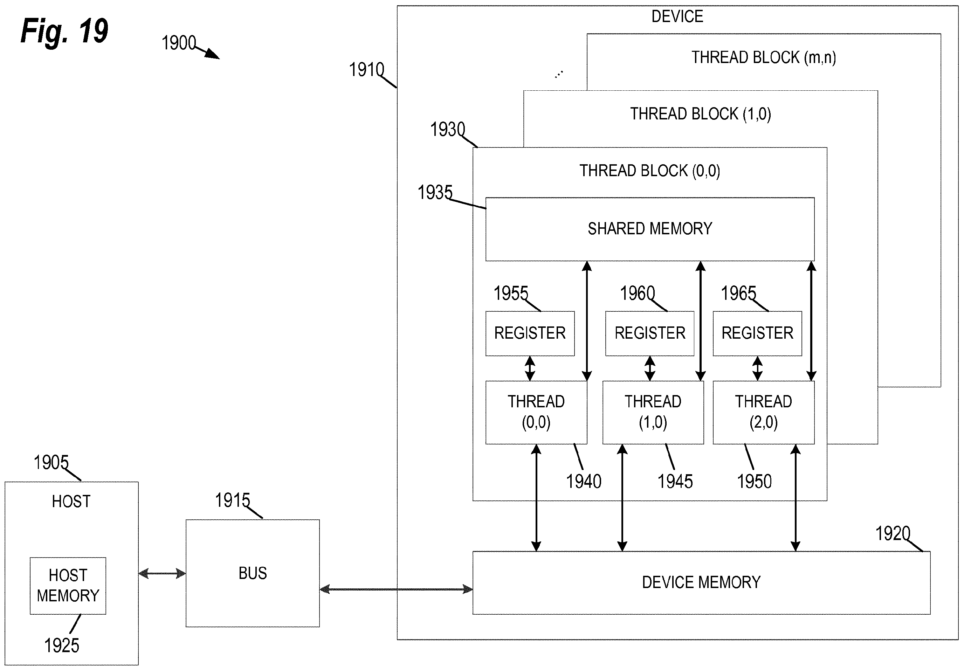

To implement off-site processing, a cloud-based computing environment may be used wherein a client device communicates with a server that hosts a highly efficient computing platform. In some embodiments, this computing platform provides parallel processing capabilities. One example of a parallel processing platform that may be utilized is illustrated below with respect to FIG. 19.

Additionally, in some embodiments, the workflows described herein can be implemented on a portable device (as an instance of wearable computing) and combined with wearable sensors for the online processing of acquired data. In other embodiments, a computing system implementing the workflows described herein may be combined with a personal assistant (e.g., Apple's SIRI), interacting with the individual in natural language (e.g., requesting the acquisition of data; or providing reminders for the actions mandated by the patient's health management plan; or providing feedback on user actions, such as a risk score for the assumption of particular foods or drinks).

As an additional supplement to the techniques described herein, once the models are executed and predictions are determined (e.g., regarding, patient outcome, suggestions for follow-up, etc.) a report can be automatically generated which presents all of the findings in a structured format (e.g., extensible markup language, Word document format, or Excel document format). The report can be part of the patient record in the hospital database.

FIG. 19 provides an example of a parallel processing platform 1900 that may be utilized to implement the ML models and other aspects of the various workflows discussed herein. This platform 1900 may be used in embodiments of the present invention where NVIDIA CUDA™ (or a similar parallel computing platform) is used. The architecture includes a host computing unit ("host") 1905 and a graphics processing unit (GPU) device ("device") 1910 connected via a bus 1915 (e.g., a PCIe bus). The host 1905 includes the central processing unit, or "CPU" (not shown in FIG. 19), and host memory 1925 accessible to the CPU. The device 1910 includes the graphics processing unit (GPU) and its associated memory 1920, referred to herein as device memory. The device memory 1920 may include various types of memory, each optimized for different memory usages. For example, in some embodiments, the device memory includes global memory, constant memory, and texture memory.

Parallel portions of a big data platform and/or big simulation platform may be executed on the platform 1900 as "device kernels" or simply "kernels." A kernel comprises parameterized code configured to perform a particular function. The parallel computing platform is configured to execute these kernels in an optimal manner across the platform 1900 based on parameters, settings, and other selections provided by the user. Additionally, in some embodiments, the parallel computing platform may include additional functionality to allow for automatic processing of kernels in an optimal manner with minimal input provided by the user.

The processing required for each kernel is performed by a grid of thread blocks (described in greater detail below). Using concurrent kernel execution, streams, and synchronization with lightweight events, the platform 1900 of FIG. 19 (or similar architectures) may be used to parallelize portions of the model based operations performed in training or utilizing the workflows discussed herein. For example, in embodiments where a convolutional neural network is used as the ML model, the platform 1900 can be used to perform operations such as forward and backward convolution, pooling, normalization, etc. with the synthetic or patient-specific data.

The device 1910 includes one or more thread blocks 1930 which represent the computation unit of the device 1910. The term thread block refers to a group of threads that can cooperate via shared memory and synchronize their execution to coordinate memory accesses. For example, in FIG. 19, threads 1940, 1945 and 1950 operate in thread block 1930 and access shared memory 1935. Depending on the parallel computing platform used, thread blocks may be organized in a grid structure. A computation or series of computations may then be mapped onto this grid. For example, in embodiments utilizing CUDA, computations may be mapped on one-, two-, or three-dimensional grids. Each grid contains multiple thread blocks, and each thread block contains multiple threads. For example, in FIG. 19, the thread blocks 1930 are organized in a two dimensional grid structure with $m+1$ rows and $n+1$ columns. Generally, threads in different thread blocks of the same grid cannot communicate or synchronize with each other. However, thread blocks in the same grid can run on the same multiprocessor within the GPU at the same time. The number of threads in each thread block may be limited by hardware or software constraints.

Continuing with reference to FIG. 19, registers 1955, 1960, and 1965 represent the fast memory available to thread block 1930. Each register is only accessible by a single thread. Thus, for example, register 1955 may only be accessed by thread 1940. Conversely, shared memory is allocated per thread block, so all threads in the block have access to the same shared memory. Thus, shared memory 1935 is designed to be accessed, in parallel, by each thread 1940, 1945, and 1950 in thread block 1930. Threads can access data in shared memory 1935 loaded from device memory 1920 by other threads within the same thread block (e.g., thread block 1930). The device memory 1920 is accessed by all blocks of the grid and may be implemented using, for example, Dynamic Random-Access Memory (DRAM).

Each thread can have one or more levels of memory access. For example, in the platform 1900 of FIG. 19, each thread may have three levels of memory access. First, each thread 1940, 1945, 1950, can read and write to its corresponding registers 1955, 1960, and 1965. Registers provide the fastest memory access to threads because there are no synchronization issues and the register is generally located close to a multiprocessor executing the thread. Second, each thread 1940, 1945, 1950 in thread block 1930, may read and write data to the shared memory 1935 corresponding to that block 1930. Generally, the time required for a thread to access shared memory exceeds that of register access due to the need to synchronize access among all the threads in the thread block. However, like the registers in the thread block, the shared memory is typically located close to the multiprocessor executing the threads. The third level of memory access allows all threads on the device 1910 to read and/or write to the device memory. Device memory requires the longest time to access because access must be synchronized across the thread blocks operating on the device. Thus, in some embodiments, data can be divided into segments using data locality techniques generally known in the art. Then, each segment can be processed in parallel using register memory, with shared and device memory only being used as necessary to combine the results to provide the results for the complete dataset.

The embodiments of the present disclosure may be implemented with any combination of hardware and software. For example, aside from parallel processing architecture presented in FIG. 19, standard computing platforms (e.g., servers, desktop computer, etc.) may be specially configured to perform the techniques discussed herein. In addition, the embodiments of the present disclosure may be included in an article of manufacture (e.g., one or more computer program products) having, for example, computer-readable, non-transitory media. The media may have embodied therein computer readable program code for providing and facilitating the mechanisms of the embodiments of the present disclosure. The article of manufacture can be included as part of a computer system or sold separately.

While various aspects and embodiments have been disclosed herein, other aspects and embodiments will be apparent to those skilled in the art. The various aspects and embodiments disclosed herein are for purposes of illustration and are not intended to be limiting. The scope of the invention is defined by the appended claims.

An executable application, as used herein, comprises code or machine readable instructions for conditioning the processor to implement predetermined functions, such as those of an operating system, a context data acquisition system or other information processing system, for example, in response to user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters.

A graphical user interface (GUI), as used herein, comprises one or more display images, generated by a display processor and enabling user interaction with a processor or other device and associated data acquisition and processing functions. The GUI also includes an executable procedure or executable application. The executable procedure or executable application conditions the display processor to generate signals representing the GUI display images. These signals are supplied to a display device which displays the image for viewing by the user. The processor, under control of an executable procedure or executable application, manipulates the GUI display images in response to signals received from the input devices. In this way, the user may interact with the display image using the input devices, enabling user interaction with the processor or other device.

The functions and process steps herein may be performed automatically or wholly or partially in response to user command. An activity (including a step) performed automatically is performed in response to one or more executable instructions or device operation without user direct initiation of the activity.

The system and processes of the figures are not exclusive. Other systems, processes and menus may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. As described herein, the various systems, subsystems, agents, managers and processes can be implemented using hardware components, software components, and/or combinations thereof. No claim element herein is to be construed under the provisions of 35 U.S.C. 112, sixth paragraph, unless the element is expressly recited using the phrase "means for."

**Claims**

1. A computer-implemented method (100) for providing a personalized evaluation of assessment of atherosclerotic plaques for a patient, the method comprising:

   training machine learning models using a database of synthetic data comprising one or more *in silico* anatomical models,
   wherein the *in silico* anatomical models are generated by:

   initializing a new *in silico* anatomical model skeleton of a coronary arterial tree anatomical model by prescribing a number of vessels at each generation of the coronary arterial tree;

defining healthy geometric information for each generation of the coronary arterial tree, wherein the healthy geometric information comprises one or more of vessel radius, a degree of tapering, and a branch length;
establishing one or more stenoses in the coronary arterial tree which modify the healthy geometric information; and
establishing plaque composition for each stenosis in the coronary arterial tree;
updating the new *in silico* anatomical model based on the modified healthy geometric information and the plaque composition for each stenosis in the coronary arterial tree; and
storing the new *in silico* anatomical model in the database of synthetic data,
wherein training the machine learning models comprises:

generating (305) a database of synthetic coronary arterial trees with plaque information *in silico,*
performing (310) a fluid solid growth computation for the *in silico* anatomical models,
extracting (315) measures of interest from the fluid solid growth computation,
extracting (320) geometric features of coronary arterial trees and plaque related features,
training (325) a data-driven surrogate model for predicting measures of interest using a machine learning method,
acquiring patient data (105, 110, 115) comprising non-invasive patient data, medical images of the patient, and blood biomarkers;
extracting (120) features of interest from the non-invasive patient data, the medical images, and the blood biomarkers;

said extracting comprising:

extracting (335) patient-specific coronary geometry from medical images,
extracting (340) geometric features of patient specific coronary arterial tree and plaque related features, and
applying (125, 330) the data driven surrogate model to the features of interest to predict one or more measures of interest related to atherosclerotic plaque, wherein the measures of interest comprise at least one of a risk of a cardiovascular event, an indication of a future screening date, a plaque composition, a future size, shape, composition and location of plaques, an in-stent restenosis, or lesions requiring sealing.

2. The method (100) according to claim 1, further comprising:

generating a report in a structured format describing the measures of interest related to atherosclerotic plaque; and
storing the report in a patient-specific medical record.

3. The method (100) according to claim 1, wherein the plaque composition for each stenosis is established by randomly selecting a particular plaque composition from a plurality of predefined plaque composition types.

4. The method (100) according to claim 3, wherein the plaque composition for each stenosis comprises a plaque material established by:

randomly selecting a center of each volume of plaque material;
randomly selecting a size and a shape for the plaque material; and
randomly selecting material proprieties for the plaque composition; and
establishing the plaque material using the center, size, shape, and material properties.

5. The method according to claim 3, wherein the plaque composition for each stenosis mimic a predefined high risk plaque composition.

6. The method (100) of claim 1, wherein the machine learning models comprise:

a first machine model trained to predict plaque formation,
a second machine model trained to predict plaque development, and
a third machine model trained to predict plaque rupture.

7. The method according to any of the preceding claims, wherein the machine learning models are applied in parallel to the features of interest.

8. The method (100) according to any of the preceding claims, wherein the machine learning models comprise:

a first machine learning model trained to predict an ischemic weight of each branch;
a second machine learning model trained to predict ischemic contribution scores,
a third machine learning model trained to predict hemodynamic measures of interest,
a fourth machine learning model trained to predict plaque related measures of interest, and
a fifth machine learning model trained to predict a risk of future cardiovascular event.

9. The method (100) according to claim 8, wherein the machine learning models are applied in a cascaded workflow that sequentially applies the machine learning models using outputs of each machine learning model as inputs for a subsequent machine learning model in the cascaded workflow.

10. The method (100) according to any of the preceding claims, further comprising:
generating a visualization of the measures of interest related to atherosclerotic plaque.

11. The method (100) of claim 10, wherein the visualization comprises a coronary artery image and the method further comprises:

    receiving a user selection of a location within the coronary artery image; and
    in response to the user selection, presenting a measure of interest corresponding to the location in the visualization.

12. A computer-implemented method for training a machine learning model to provide a personalized evaluation of assessment of atherosclerotic plaques for a patient, the method comprising:

    training machine learning models using a database of synthetic data comprising one or more *in silico* anatomical models,
    wherein the *in silico* anatomical models are generated by:

        initializing a new *in silico* anatomical model skeleton of a coronary arterial tree anatomical model by prescribing a number of vessels at each generation of the coronary arterial tree;
        defining healthy geometric information for each generation of the coronary arterial tree, wherein the healthy geometric information comprises one or more of vessel radius, a degree of tapering, and a branch length;
        establishing one or more stenoses in the coronary arterial tree which modify the healthy geometric information; and
        establishing plaque composition for each stenosis in the coronary arterial tree;
        updating the new *in silico* anatomical model based on the modified healthy geometric information and the plaque composition for each stenosis in the coronary arterial tree; and
    storing the new *in silico* anatomical model in the database of synthetic data,
    wherein training the machine learning models comprises:

        generating (305) a database of synthetic coronary arterial trees with plaque information *in silico,*
        performing (310) a fluid solid growth computation for the *in silico* anatomical models,
        extracting (315) measures of interest from the fluid solid growth computation,
        extracting (320) geometric features of coronary arterial trees and plaque related features,
        training (325) a data-driven surrogate model for predicting measures of interest using a machine learning method,

13. The method according to claim 12, further comprising:
applying the one or more machine learning models to the features of interest to generate predicted measurements related to atherosclerotic plaque.

14. The method according to claim 13, further comprising:

    receiving patient-specific measurements related to atherosclerotic plaque;
    comparing the patient-specific measurements related to atherosclerotic plaque to the predicted measurements related to atherosclerotic plaque;
    if the patient-specific measurements do not match the predicted measurements, retaining the one or more machine learning models using the patient-specific measurements.

15. The method according to any of the preceding claims 12 to 14, further comprising:

    receiving a specification of input uncertainties associated with each of the one or more of *in silico* anatomical models;
    determining a confidence interval for each of the measures of interest;
    using the confidence interval and the *in silico* anatomical models to train the one or more machine learning models to predict confidence intervals of the predicted measurements related to atherosclerotic plaque based on geometric features of anatomical models.

16. The method according to claim 1, further comprising:

generating a report in a structured format describing the measures of interest related to atherosclerotic plaque; and
storing the report in a patient-specific medical record.

## Patentansprüche

1. Computer-implementiertes Verfahren (100) zum Bereitstellen einer personalisierten Bewertung der Beurteilung von atherosklerotischen Plaques für einen Patienten, wobei das Verfahren umfasst:

Training von Modellen des maschinellen Lernens unter Verwendung einer Datenbank mit synthetischen Daten, die ein oder mehrere anatomische *In-silico-Modelle* umfassen,
wobei die anatomischen *In-silico Modelle* erzeugt werden durch:

Initialisierung eines neuen anatomischen *in silico* Modellskeletts eines anatomischen Modells des koronaren Arterienbaums durch Vorgabe einer Anzahl von Gefäßen bei jeder Generation des koronaren Arterienbaums;
Definieren gesunder geometrischer Informationen für jede Generation des koronaren Arterienbaums, wobei die gesunden geometrischen Informationen einen oder mehrere Gefäßradien, einen Verjüngungsgrad und eine Verzweigungslänge umfassen;
Feststellen einer oder mehrerer Stenosen im koronaren Arterienbaum, die die gesunde geometrische Information verändern; und
Ermitteln der Plaque-Zusammensetzung für jede Stenose im koronaren Arterienbaum;
Aktualisieren des neuen anatomischen *In-silico-Modells* auf der Grundlage der modifizierten gesunden geometrischen Informationen und der Plaque-Zusammensetzung für jede Stenose im koronaren Arterienbaum; und
Speichern des neuen anatomischen *in silico* Modells in der Datenbank der synthetischen Daten,
wobei das Training der maschinellen Lernmodelle umfasst:

Erstellen (305) der Datenbank synthetischer koronarer Arterienbäume mit Plaque-Informationen *in silico,*
Durchführen (310) einer Flüssigkeits-Festkörper-Wachstumsberechnung für die anatomischen *in silico-Modelle ,*
Extraktion von (315) interessierenden Maßen aus der Berechnung des Flüssigkeits-Festkörper-Wachstums,
Extraktion (320) geometrischer Merkmale von koronaren Arterienbäumen und plaquebezogener Merkmale,
Training (325) eines datengesteuerten Surrogatmodells für die Vorhersage der interessierenden Maße unter Verwendung einer maschinellen Lernmethode,
Erfassung von Patientendaten (105, 110, 115), die nicht-invasive Patientendaten, medizinische Bilder des Patienten und Blutbiomarker umfassen;
Extrahieren (120) von interessierenden Merkmalen aus den nicht-invasiven Patientendaten, den medizinischen Bildern und den Blut-Biomarkern;
wobei das Extrahieren umfasst:

Extraktion (335) der patientenspezifischen Koronargeometrie aus medizinischen Bildern,
Extraktion (340) geometrischer Merkmale des patientenspezifischen koronaren Arterienbaums und plaquebezogener Merkmale, und
Anwenden (125, 330) des datengesteuerten Surrogatmodells auf die interessierenden Merkmale zur Vorhersage eines oder mehrerer interessierender Maße in Bezug auf atherosklerotische Plaques, wobei die interessierenden Maße mindestens eines der folgenden Merkmale umfassen: das Risiko eines kardiovaskulären Ereignisses, einen Hinweis auf ein zukünftiges Screening-Datum, eine Plaque-Zusammensetzung, eine zukünftige Größe, Form, Zusammensetzung und Lage von Plaques, eine In-Stent-Restenose oder Läsionen, die eine Versiegelung erfordern.

2. Verfahren (100) nach Anspruch 1, ferner umfassend:

Erzeugen eines Berichts in einem strukturierten Format, der die interessierenden Maße in Bezug auf die atherosklerotische Plaque beschreibt; und
Speichern des Berichts in einer patientenbezogenen Krankenakte.

**3.** Verfahren (100) nach Anspruch 1, wobei die Plaque-Zusammensetzung für jede Stenose durch zufällige Auswahl einer bestimmten Plaque-Zusammensetzung aus einer Vielzahl von vordefinierten Plaque-Zusammensetzungstypen bestimmt wird.

**4.** Verfahren (100) nach Anspruch 3, wobei die Plaque-Zusammensetzung für jede Stenose ein Plaque-Material umfasst, **gekennzeichnet durch**:

zufällige Auswahl der Mitte eines jeden Volumens von Plaque-Material;
zufällige Auswahl einer Größe und einer Form für das Plaque-Materials; und
zufällige Auswahl von Materialeigenschaften für die Plaque-Zusammensetzung; und
Festlegung des Plaque-Materials anhand des Zentrums, der Größe, der Form und der Materialeigenschaften.

**5.** Verfahren nach Anspruch 3, wobei die Plaque-Zusammensetzung für jede Stenose eine vordefinierte Hochrisiko-Plaque-Zusammensetzung nachahmt.

**6.** Verfahren (100) nach Anspruch 1, wobei die maschinellen Lernmodelle umfassen:

ein erstes Maschinenmodell, das für die Vorhersage der Plaquebildung trainiert wurde,
ein zweites Maschinenmodell, das für die Vorhersage der Plaqueentwicklung trainiert wurde, und
ein drittes Maschinenmodell, das für die Vorhersage von Plaquerupturen trainiert wurde.

**7.** Verfahren nach einem der vorhergehenden Ansprüche, wobei die maschinellen Lernmodelle parallel auf die interessierenden Merkmale angewendet werden.

**8.** Verfahren (100) nach einem der vorhergehenden Ansprüche, wobei die maschinellen Lernmodelle umfassen:

ein erstes maschinelles Lernmodell, das für die Vorhersage eines ischämischen Gewichts jedes Zweigs trainiert wurde;
ein zweites maschinelles Lernmodell, das für die Vorhersage von ischämischen Beiträgen trainiert wurde,
ein drittes maschinelles Lernmodell, das für die Vorhersage der interessierenden hämodynamischen Größen trainiert wurde,
ein viertes maschinelles Lernmodell, das für die Vorhersage von Plaque-bezogenen interessierenden Maßen trainiert wurde, und
ein fünftes Modell des maschinellen Lernens, das für die Vorhersage des Risikos eines künftigen kardiovaskulären Ereignisses trainiert wurde.

**9.** Verfahren (100) nach Anspruch 8, wobei die maschinellen Lernmodelle in einem kaskadierten Arbeitsablauf angewendet werden, der die maschinellen Lernmodelle sequentiell anwendet, wobei Ausgaben jedes maschinellen Lernmodells als Eingaben für ein nachfolgendes maschinelles Lernmodell in dem kaskadierten Arbeitsablauf verwendet werden.

**10.** Verfahren (100) nach einem der vorhergehenden Ansprüche, das ferner umfasst:
eine Visualisierung der für die atherosklerotische Plaque interessierenden Maße zu erstellen.

**11.** Verfahren (100) nach Anspruch 10, wobei die Visualisierung ein Koronararterienbild umfasst und das Verfahren ferner umfasst:

Empfangen einer Benutzerauswahl einer Stelle innerhalb des Koronararterienbildes; und
Darstellen, als Reaktion auf die Auswahl des Benutzers, eines interessierenden Maßes korrespondierend zu dem Ort der Visualisierung.

**12.** Computerimplementiertes Verfahren zum Trainieren eines maschinellen Lernmodells, um eine personalisierte Bewertung der Beurteilung von atherosklerotischen Plaques für einen Patienten bereitzustellen, wobei das Verfahren umfasst:

Training von Modellen des maschinellen Lernens unter Verwendung einer Datenbank mit synthetischen Daten, die ein oder mehrere anatomische *In-silico-Modelle* umfassen,
wobei die anatomischen *In-silico Modelle* erzeugt werden durch:

Initialisierung eines neuen anatomischen *in silico* Modellskeletts eines anatomischen Modells des koronaren Arterienbaums durch Vorgabe einer Anzahl von Gefäßen bei jeder Generation des koronaren Arterienbaums;
Definieren gesunder geometrischer Informationen für jede Generation des koronaren Arterienbaums, wobei die gesunden geometrischen Informationen einen oder mehrere Gefäßradien, einen Verjüngungsgrad und eine Verzweigungslänge umfassen;
Feststellen einer oder mehrerer Stenosen im koronaren Arterienbaum, die die gesunde geometrische Information verändern; und
Ermitteln der Plaque-Zusammensetzung für jede Stenose im koronaren Arterienbaum;
Aktualisieren des neuen anatomischen *In-silico-Modells* auf der Grundlage der modifizierten gesunden geometrischen Informationen und der Plaque-Zusammensetzung für jede Stenose im koronaren Arterienbaum; und
Speichern des neuen anatomischen *in silico* Modells in der Datenbank der synthetischen Daten,
wobei das Training der maschinellen Lernmodelle umfasst:

> Erstellen (305) einer Datenbank mit synthetischen koronaren Arterienbäumen mit Plaque-Informationen *in silico,*
> Durchführen (310) einer Flüssigkeits-Festkörper-Wachstumsberechnung für die anatomischen *in silico-Modelle ,*
> Extraktion von (315) interessierenden Maßen aus der Berechnung des Flüssigkeits-Festkörper-Wachstums,
> Extraktion (320) geometrischer Merkmale von koronaren Arterienbäumen und plaquebezogener Merkmale,
> Training (325) eines datengesteuerten Surrogatmodells für die Vorhersage der interessierenden Maße unter Verwendung einer maschinellen Lernmethode,

**13.** Verfahren nach Anspruch 12 umfasst ferner:
Anwendung des einen oder der mehreren maschinellen Lernmodelle auf die interessierenden Merkmale, um vorhergesagte Messungen in Bezug auf atherosklerotische Plaque zu erzeugen.

**14.** Verfahren nach Anspruch 13 umfasst ferner:

> Empfangen patientenbezogener Messungen im Zusammenhang mit atherosklerotischen Plaques;
> Vergleichen der patientenspezifischen Messungen in Bezug auf atherosklerotische Plaques mit den vorhergesagten Messungen in Bezug auf atherosklerotische Plaques;
> wenn die patientenspezifischen Messungen nicht mit den vorhergesagten Messungen übereinstimmen, Beibehaltung des einen oder der mehreren maschinellen Lernmodelle unter Verwendung der patientenspezifischen Messungen.

**15.** Verfahren nach einem der vorangehenden Ansprüche 12 bis 14, das ferner umfasst:

> Empfangen einer Spezifikation von Eingabeunsicherheiten, die mit jedem des einen oder der mehreren anatomischen *In-silico-Modelle* verbunden sind;
Bestimmung eines Konfidenzintervalls für jedes der interessierenden Maße;
Verwendung des Konfidenzintervalls und der anatomischen *In-silico-Modelle* zum Trainieren des einen oder der mehreren maschinellen Lernmodelle zur Vorhersage von Konfidenzintervallen der vorhergesagten Messungen in Bezug auf atherosklerotische Plaque auf der Grundlage geometrischer Merkmale anatomischer Modelle.

**16.** Verfahren nach Anspruch 1 ferner umfassend:

> Erzeugen eines Berichts in einem strukturierten Format, der die interessierenden Maße in Bezug auf die atherosklerotische Plaque beschreibt; und
> Speichern des Berichts in einer patientenbezogenen Krankenakte.

**Revendications**

**1.** Méthode mise en oeuvre par ordinateur (100) pour fournir une évaluation personnalisée de l'évaluation des plaques d'athérosclérose pour un patient, la méthode comprenant :

> la formation de modèles d'apprentissage automatique à l'aide d'une base de données synthétiques comprenant un ou plusieurs modèles anatomiques *in silico ,*
> dans lequel les modèles anatomiques *in silico* sont générés par :

>> initialiser un nouveau squelette de modèle anatomique *in silico* d'un modèle anatomique d'arbre artériel coronaire en prescrivant un nombre de vaisseaux à chaque génération de l'arbre artériel coronaire ;
>> définir des informations géométriques saines pour chaque génération de l'arbre artériel coronaire, les informations géométriques saines comprenant un ou plusieurs des éléments suivants : rayon du vaisseau, degré d'amincissement et longueur de la branche ;
>> établir une ou plusieurs sténoses dans l'arbre artériel coronaire qui modifient les informations géométriques saines ; et
>> établir la composition de la plaque pour chaque sténose de l'arbre artériel coronaire ;
>> mettre à jour le nouveau modèle anatomique *in silico* sur la base des informations géométriques saines modifiées et de la composition de la plaque pour chaque sténose de l'arbre artériel coronaire ; et
stocker le nouveau modèle anatomique *in silico* dans la base de données synthétiques,

dans lequel l'entraînement des modèles d'apprentissage automatique comprend générer (305) la base de données d'arbres artériels coronaires synthétiques avec des informations sur la plaque *in silico,*

effectuer (310) un calcul de croissance solide fluide pour les modèles anatomiques *in silico ,*

l'extraction (315) de mesures d'intérêt à partir du calcul de la croissance des solides fluides,

l'extraction (320) des caractéristiques géométriques des arbres artériels coronaires et des caractéristiques liées à la plaque,

la formation (325) d'un modèle de substitution basé sur des données pour prédire les mesures d'intérêt à l'aide d'une méthode d'apprentissage automatique,

l'acquisition de données sur le patient (105, 110, 115) comprenant des données non invasives sur le patient, des images médicales du patient et des biomarqueurs sanguins ;

l'extraction (120) de caractéristiques intéressantes à partir des données non invasives du patient, des images médicales et des biomarqueurs sanguins ;

ladite extraction comprenant :

> l'extraction (335) de la géométrie coronaire spécifique au patient à partir d'images médicales,
>
> l'extraction (340) des caractéristiques géométriques de l'arbre artériel coronaire spécifique au patient et des caractéristiques liées à la plaque, et
>
> appliquer (125, 330) le modèle de substitution piloté par les données aux caractéristiques d'intérêt pour prédire une ou plusieurs mesures d'intérêt liées à la plaque d'athérosclérose, les mesures d'intérêt comprenant au moins l'un des éléments suivants : risque d'événement cardiovasculaire, indication d'une future date de dépistage, composition de la plaque, taille, forme, composition et emplacement futurs des plaques, resténose du stent ou lésions nécessitant un scellement.

2. La méthode (100) selon la revendication 1, comprenant en outre :

> générer un rapport dans un format structuré décrivant les mesures d'intérêt liées à la plaque d'athérosclérose ; et
stocker le rapport dans un dossier médical spécifique au patient.

3. La méthode (100) selon la revendication 1, dans laquelle la composition de la plaque pour chaque sténose est établie en sélectionnant au hasard une composition de plaque particulière parmi une pluralité de types de composition de plaque prédéfinis.

4. La méthode (100) selon la revendication 3, dans laquelle la composition de la plaque pour chaque sténose comprend un matériau de plaque établi par :

> sélection aléatoire d'un centre de chaque volume de plaque ;
> sélection aléatoire d'une taille et d'une forme pour le matériau de la plaque ; et
> sélection aléatoire des propriétés des matériaux pour la composition de la plaque ; et
> établir le matériau de la plaque en utilisant le centre, la taille, la forme et les propriétés du matériau.

5. La méthode selon la revendication 3, dans laquelle la composition de la plaque pour chaque sténose imite une composition prédéfinie de plaque à haut risque.

6. La méthode (100) de la revendication 1, dans laquelle les modèles d'apprentissage automatique comprennent :

> un premier modèle machine entraîné à prédire la formation de la plaque,
> un deuxième modèle machine entraîné à prédire le développement de la plaque, et
> un troisième modèle de machine entraîné à prédire la rupture de la plaque.

7. La méthode selon l'une des revendications précédentes, dans laquelle les modèles d'apprentissage automatique sont appliqués en parallèle aux caractéristiques d'intérêt.

8. La méthode (100) selon l'une des revendications précédentes, dans laquelle les modèles d'apprentissage automatique comprennent :

> un premier modèle d'apprentissage automatique formé pour prédire le poids ischémique de chaque branche ;
> un deuxième modèle d'apprentissage automatique entraîné à prédire les scores de contribution ischémique,
> un troisième modèle d'apprentissage automatique formé pour prédire les mesures hémodynamiques d'intérêt,
un quatrième modèle d'apprentissage automatique entraîné à prédire les mesures d'intérêt liées à la plaque, et
un cinquième modèle d'apprentissage automatique formé pour prédire un risque d'événement cardiovasculaire futur.

9. La méthode (100) selon la revendication 8, dans laquelle les modèles d'apprentissage automatique sont appliqués dans un flux de travail en cascade qui applique séquentiellement les modèles d'apprentissage automatique en utilisant les résultats de chaque modèle d'apprentissage automatique comme entrées pour un modèle d'apprentissage automatique ultérieur dans le flux de travail en cascade.

10. La méthode (100) selon l'une des revendications précédentes, comprenant en outre :
génerer une visualisation des mesures d'intérêt liées à la plaque d'athérosclérose.

11. La méthode (100) de la revendication 10, dans laquelle la visualisation comprend une image d'artère coronaire et la méthode comprend en outre :

recevoir une sélection de l'utilisateur d'un emplacement dans l'image de l'artère coronaire ; et
en réponse à la sélection de l'utilisateur, présenter une mesure d'intérêt correspondant à l'emplacement dans la visualisation.

12. Méthode mise en oeuvre par ordinateur pour former un modèle d'apprentissage automatique afin de fournir une évaluation personnalisée de l'évaluation des plaques d'athérosclérose pour un patient, la méthode comprenant :

la formation de modèles d'apprentissage automatique à l'aide d'une base de données synthétiques comprenant un ou plusieurs modèles anatomiques *in silico* ,
dans lequel les modèles anatomiques *in silico* sont générés par :

initialiser un nouveau squelette de modèle anatomique *in silico* d'un modèle anatomique d'arbre artériel coronaire en prescrivant un nombre de vaisseaux à chaque génération de l'arbre artériel coronaire ;
définir des informations géométriques saines pour chaque génération de l'arbre artériel coronaire, les informations géométriques saines comprenant un ou plusieurs des éléments suivants : rayon du vaisseau, degré d'amincissement et longueur de la branche ;
établir une ou plusieurs sténoses dans l'arbre artériel coronaire qui modifient les informations géométriques saines ; et
établir la composition de la plaque pour chaque sténose de l'arbre artériel coronaire ;
mettre à jour le nouveau modèle anatomique *in silico* sur la base des informations géométriques saines modifiées et de la composition de la plaque pour chaque sténose de l'arbre artériel coronaire ; et
stocker le nouveau modèle anatomique *in silico* dans la base de données synthétiques,
dans lequel l'entraînement des modèles d'apprentissage automatique comprend générer (305) une base de données d'arbres artériels coronaires synthétiques avec des informations sur les plaques *in silico,*
effectuer (310) un calcul de croissance solide fluide pour les modèles anatomiques *in silico* ,
l'extraction (315) de mesures d'intérêt à partir du calcul de la croissance des solides fluides,
l'extraction (320) des caractéristiques géométriques des arbres artériels coronaires et des caractéristiques liées à la plaque,
la formation (325) d'un modèle de substitution basé sur des données pour prédire les mesures d'intérêt à l'aide d'une méthode d'apprentissage automatique,

13. La méthode selon la revendication 12, comprenant en outre :
appliquer un ou plusieurs modèles d'apprentissage automatique aux caractéristiques d'intérêt pour générer des mesures prédites liées à la plaque d'athérosclérose.

14. La méthode selon la revendication 13, comprenant en outre :

recevoir des mesures spécifiques au patient liées à la plaque d'athérosclérose ;
comparer les mesures spécifiques au patient relatives à la plaque d'athérosclérose aux mesures prédites relatives à la plaque d'athérosclérose ;
si les mesures spécifiques au patient ne correspondent pas aux mesures prédites, conserver le ou les modèles d'apprentissage automatique en utilisant les mesures spécifiques au patient.

15. La méthode selon l'une des revendications précédentes 12 à 14, comprenant en outre :

recevoir une spécification des incertitudes d'entrée associées à chacun des modèles anatomiques *in silico* ;

déterminer un intervalle de confiance pour chacune des mesures d'intérêt ;
utiliser l'intervalle de confiance et les modèles anatomiques *in silico* pour entraîner un ou plusieurs modèles d'apprentissage automatique à prédire les intervalles de confiance des mesures prédites liées à la plaque d'athérosclérose sur la base des caractéristiques géométriques des modèles anatomiques.

16. La méthode selon la revendication 1, comprenant en outre :

générer un rapport dans un format structuré décrivant les mesures d'intérêt liées à la plaque d'athérosclérose ; et
stocker le rapport dans un dossier médical spécifique au patient.

*Fig. 1*

200

**205**

Database containing non-invasive patient data, medical images of patients, blood biomarkers, data extracted from in vitro experiments and outcome measure(s) of interest

**215**

Extract outcome measure(s) of interest

**210**

Extract features from patient-specific data

**220**

Train one or more data-driven surrogate model for predicting outcome measures of interest using machine learning algorithms

*Fig. 2*

**305**
Generate a database of synthetic coronary arterial trees with plaque information (*in silico* or *in vitro* anatomical models)

**335**
Extract patient-specific coronary geometry from medical images (X-ray Angiography, CTA, etc.)

**300**

**310**
Perform FSG computation for in silico models or flow experiment for in vitro models

**320**
Extract geometric features of coronary arterial trees and plaque related features

**340**
Extract geometric features of patient-specific coronary arterial tree and plaque related features

**315**
Extract measures of interest from FSG computations / flow experiments (plaque formation, progression, rupture, etc.)

**325**
Train a data-driven surrogate model for predicting measures of interest using a machine learning method

**330**
Use surrogate model to predict patient-specific measures of interest (plaque formation, progression, rupture, etc.)

*Fig. 3*

400

```
┌─────────────────────────────────┐
│              405                │
│  Initialize skeleton of coronary │
│           geometry               │
└─────────────────────────────────┘
                 │
                 ▼
┌─────────────────────────────────┐
│              410                │
│ Prescribe geometric information  │
│   for each generation of the     │
│          vessel tree             │
└─────────────────────────────────┘
                 │
                 ▼
┌─────────────────────────────────┐
│              415                │
│   Generate stenoses in the       │
│       coronary vessels           │
└─────────────────────────────────┘
                 │
                 ▼
┌─────────────────────────────────┐
│              420                │
│ Determine plaque composition of  │
│  all stenoses generated at step  │
│             415                  │
└─────────────────────────────────┘
```

*Fig. 4A*

**Fig. 4B**

**Fig. 4C**

Length

Stenosis center

Length with min. radius

Radius reduction

Tapering

**Fig. 4D**

Fibrous tissue

Necrotic core

Lipid pool

Calcificatio n

**Fig. 4E**

Fig. 5

**Geometry** →

**Plaque morphology** →

Cascaded ML workflow

605
ML-based prediction of ischemic weight

610
Geometric / Plaque morphology features

615
ML-based prediction of ischemic contribution scores

620
ML-based prediction of Reynolds number

625
ML-based prediction of hemodynamic measures of interest (flow, pressure, WSS, TAWSS, OSI, relative residence time, etc.)

630
ML-based prediction of plaque related measures of interest (formation, evolution, rupture)

635
ML-based prediction of cardiovascular event risk score

600

*Fig. 6*

**705**
Generate a database of synthetic coronary arterial trees with plaque information required for FSG simulations

**710**
Specify input uncertainties (vascular geometry, plaque geometry, material properties, etc.)

**715**
Extract geometric features of vascular anatomical models and other features of interest

**720**
Perform physics based computation for in silico models (using uncertain input variables)

**725**
Determine confidence interval of plaque related measure of interest

**730**
Train a data-driven surrogate model for predicting confidence interval of plaque related measure of interest using a machine learning method

**735**
Use surrogate model to predict confidence interval of plaque related measure of interest

**740**
Extract patient-specific vascular anatomical model (CTA, XA, MRI, etc.)

**745**
Estimate uncertainty of input data automatically or from user input

**750**
Extract geometric features and other features of interest from the patient-specific vascular anatomical models

**700**

*Fig. 7*

800

**805**
Identify patient-specific case with bad match between predicted and measured plaque related output

**810**
Determine reason for bad match (e.g. specific feature values which were not present in the database of synthetic cases)

**815**
Generate new synthetic cases with feature values similar to the ones encountered for the patient-specific case with bad match

**820**
Retrain machine learning algorithm on entire database

*Fig. 8*

**900**

```
┌─────────────────────────────────────────────┐
│                     905                       │
│  Perform in vitro studies related to plaque   │
│        formation, evolution and rupture       │
└─────────────────────────────────────────────┘
```

```
┌──────────────────┐        ┌──────────────────┐
│       910        │        │       915        │
│ Extract features │        │ Extract plaque   │
│ of interest      │        │ related measures │
│ (geometry, flow, │        │ of interest      │
│ plaque, etc.)    │        │                  │
└──────────────────┘        └──────────────────┘
```

```
┌─────────────────────────────────────────────┐
│                     920                       │
│  Train one or more data-driven surrogate      │
│  model for predicting plaque related          │
│  measures of interest using machine           │
│  learning algorithms                          │
└─────────────────────────────────────────────┘
```

*Fig. 9*

*Fig. 10*

**1105**
Perform in vitro studies related to plaque formation, evolution and rupture

**1100**

**1110**
Apply a nonlinear dynamical systems discovery method to derive plaque related governing equations

**1115**
Perform synthetic in silico experiments to cover a broad range of physical states

**1120**
Train one or more data-driven surrogate model for predicting plaque related measures of interest using machine learning algorithms

*Fig. 11*

Plaque composition — Fibrous tissue, Necrotic core, Lipid pool, Calcification

Plaque appearance on CTA — Fibrous tissue, Necrotic core, Lipid pool, Calcification

Fig. 12

```
                                        ┌─────────────────────┐
                                        │        1305         │
                                        │ Prepare plaque      │
                                        │ samples             │──────┐
                                        │ (tissue engineering,│      │
                                        │ extracted           │      │         ← 1300
                                        │ from cadavers, etc.)│      │
                                        └─────────────────────┘      │
                                                   │                 │
                                                   ▼                 │
                                        ┌─────────────────────┐      │
                                        │        1310         │      │
                                        │ Acquire medical     │      │
                                        │ images of plaque    │      │
                                        │ samples (CTA, MRI,  │      │
                                        │ XA, US, etc.)       │      │
                                        └─────────────────────┘      │
                                                   │                 ▼
                                                   ▼        ┌─────────────────────┐
                                        ┌──────────────────┐│        1320         │
                                        │      1315        ││ Extract measures of │
                                        │ Extract features ││ interest from       │
                                        │ of interest from ││ plaque samples      │
                                        │ medical images   │└─────────────────────┘
                                        └──────────────────┘          │
                                                   │                  │
                                                   ▼                  │
                                        ┌──────────────────┐          │
                                        │      1325        │◄─────────┘
                                        │ Train one or     │
                                        │ more data-driven │
                                        │ surrogate models │
                                        │ for performing   │
                                        │ plaque histology │
                                        └──────────────────┘
```

*Fig. 13*

EP 3 404 667 B1

1400

1405
Identify vessel lumen (manually, semi-automatically or automatically)

1410
Manually identify plaque (possibly separated into different plaque components)

1415
Train one or more data-driven surrogate models for automatically identifying the plaque on medical images using machine learning algorithms

*Fig. 14*

1505
Generate a database of synthetic coronary arterial trees where lesions are sealed

1530
Extract patient-specific coronary geometry from medical images (X-ray Angiography, CTA, etc.)

1510
Perform FSG computation for in silico models or flow experiment for in vitro models

1515
Extract modified features of coronary arterial trees

1535
Extract modified features of patient-specific coronary arterial tree

1520
Extract plaque related measures of interest from FSG computations / flow experiments

1523
Train a data-driven surrogate model for predicting measures of interest using a machine learning method

1525
Use surrogate model to predict patient-specific plaque related measures of interest

1500

*Fig. 15*

**1610**
Extract patient-specific geometry from medical images, non-invasive patient data, blood biomarkers

**1615**
Extract geometric and plaque related features of interest

**1620**
Use machine learning model to predict the effect of a treatment plan

**1605**
Database containing patient-specific features and longitudinal data related to the effect of treatment plans

**1625**
Train machine learning model for predicting the effect of a treatment plan

1600

*Fig. 16*

fibrous tissue 15%, necrotic core 10%, lipid pool 55%, calcification 20%

Future screening date: **mm/dd/ 20yy**
Risk of cardiovascular event: **5%**
Lesion requiring sealing: **no**
Medical therapy: **statins, fibrates**

*Fig. 17*

*Fig. 18*

# Fig. 19

1900 ➔

**REFERENCES CITED IN THE DESCRIPTION**

**Patent documents cited in the description**

- US 2016148371 A1
- US 2017018081 A1

- US 9349178 B, Itu